# EUROPÄISCHE PATENTSCHRIFT

(11) **EP 1 409 441 B1**
(45) Veröffentlichungstag und Bekanntmachung des Hinweises auf die Patenterteilung: **18.11.2009**
(21) Anmeldenummer: 02767236.9
(22) Anmeldetag: 19.07.2002
(51) Int. Cl.: C07C 25/22, C07C 43/225, C07C 49/697, C07C 49/755, C07D 307/79, C09K 19/32, C09K 19/34, G02F 1/13

(54) **INDANVERBINDUNGEN MIT NEGATIVER DIELEKTRISCHER ANISOTROPIE**
INDANE COMPOUNDS WITH NEGATIVE DIELECTRIC ANISOTROPY
COMPOSES DE L'INDANE A ANISOTROPIE DIELECTRIQUE NEGATIVE

(30) Priorität: 20.07.2001 DE 10135499
(43) Veröffentlichungstag der Anmeldung: 21.04.2004
(73) Patentinhaber: Merck Patent GmbH, 64293 Darmstadt (DE)
(72) Erfinder: REIFFENRATH, Volker, 64380 Ro dorf (DE); HECKMEIER, Michael, 69502 Hemsbach (DE); BREMER, Matthias, 64295 Darmstadt (DE)
(74) Vertreter: Isenbruck, Günter
(86) Internationale Anmeldenummer: PCT/EP2002/008085
(87) Internationale Veröffentlichungsnummer: WO 2003/010120

(56) Entgegenhaltungen:
- EP-A- 1 350 780
- DE-A- 4 303 634
- DE-A- 19 840 447
- DE-A- 19 900 517
- DE-A- 19 909 761
- DE-C- 279 864
- JP-A- 08 157 463
- JP-A- 08 169 883
- US-A- 2 447 099
- WATANABE M ET AL: "A REGIOSELECTIVE LITHIATION OF ORTHO-CRESOLS USING THE BIS (DIMETHYLAMINO)PHOSPHORYL GROUP AS A DIRECTING GROUP: GENERAL SYNTHESIS OF 2,3-DIHYDROBENZO[B]FURANS INCLUDING NATURALLY OCCURRING NEOLIGNANS" CHEMICAL AND PHARMACEUTICAL BULLETIN, PHARMACEUTICAL SOCIETY OF JAPAN. TOKYO, JP, Bd. 39, Nr. 12, Dezember 1991 (1991-12), Seiten 3123-3131, XP002934709 ISSN: 0009-2363
- BAVIN P M G ET AL: "COMPOUNDS AFFECTING THE CENTRAL NERVOUS SYSTEM. V. SUBSTITUTED 3-DIALKYLAMINOALKYLINDENES" JOURNAL OF MEDICINAL CHEMISTRY, AMERICAN CHEMICAL SOCIETY. WASHINGTON, US, Bd. 12, Nr. 3, 1. Mai 1969 (1969-05-01), Seiten 513-516, XP002020326 ISSN: 0022-2623
- GATES B D ET AL: "MECHANISTIC ASPECTS AND SYNTHETIC APPLICATIONS OF THE ELECTROCHEMICAL AND IODOBENZENE BIS(TRIFLUOROACETATE) OXIDATIVE 1,3-CYCLOADDITIONS OF PHENOLS AND ELECTRON-RICH STYRENE DERIVATIVES" JOURNAL OF ORGANIC CHEMISTRY, AMERICAN CHEMICAL SOCIETY. EASTON, US, Bd. 57, 1992, Seiten 2135-2143, XP002934710 ISSN: 0022-3263
- GREIFENSTEIN L G ET AL: "Response of Acidity and Magnetic Resonance Properties to Aryl Substitution in Carbon Acids and Derived Carbanions: 2- and 3-Arylindenes" JOURNAL OF ORGANIC CHEMISTRY, AMERICAN CHEMICAL SOCIETY. EASTON, US, Bd. 25, Nr. 46, 1981, Seiten 5125-5132, XP002075253 ISSN: 0022-3263
- LEDNICER, D. ET AL.: "Mammalian antifertility agents. I. Derivatives of 2,3-diphenylindenes" J. MED. CHEM., Bd. 8, 1965, Seiten 52-57, XP002220475
- SUZUKI, T. ET AL.: "Superacid-catalyzed electrocyclization of 1-phenyl-2-propen-1-ones to 1-indanones. Kinetic and theoretical studies of electrocyclization of oxonium-carbenium dications" J. AM. CHEM. SOC., Bd. 119, Nr. 29, 1997, Seiten 6774-6780, XP002220476
- HURD, C.D. ET AL.: "Directed ring closure in the synthesis of chromans and coumarans from o-allylphenols" J. ORG. CHEM., Bd. 5, 1940, Seiten 212-222, XP002220477
- LAROCK, R.C. ET AL.: "Palladium-catalyzed heteroannulation of 1,3-dienes by functionally substituted aryl halides" J. ORG. CHEM., Bd. 55, Nr. 11, 1990, Seiten 3447-3450, XP002220478
- JHA, G. ET AL.: "Synthesis of indeno[2,1-c]isocoumarins & indeno[2,1-c]isoquinolones" INDIAN J. CHEM., Bd. 24B, 1985, Seiten 440-444, XP009000459
- BICKERT, P. ET AL.: "Phane des 1,5- und 1,7-Dihydro-s-indacens" ANGEW. CHEM., Bd. 94, Nr. 4, 1982, Seite 308 XP009000469
- RUGGLI, P. ET AL.: "Über die Synthese eines linearen Dioxo-dicyclopenteno-benzols" HELV. CHIM. ACTA, Bd. 30, 1947, Seiten 2048-2057, XP009000457

## Beschreibung

Die Erfindung betrifft Indanverbindungen mit negativem Δε

Flüssigkristalle haben ein breites Anwendungsfeld gefunden, seitdem vor etwa 30 Jahren die ersten kommerziell anwendbaren flüssigkristallinen Verbindungen aufgefunden wurden. Bekannte Anwendungsgebiete sind insbesondere Anzeigedisplays für Uhren oder Taschenrechner, oder große Anzeigetafeln, wie sie in Bahnhöfen, Flughäfen und Sportarenen verwendet werden. Weitere Anwendungsgebiete sind Displays von tragbaren Computern oder Navigationssystemen sowie Videoapplikationen. Insbesondere für die zuletzt genannten Anwendungen werden hohe Anforderungen an Schaltzeiten und den Kontrast der Abbildungen gestellt.

Die räumliche Ordnung der Moleküle in einem Flüssigkristall bewirkt, daß viele seiner Eigenschaften richtungsabhängig sind. Von Bedeutung für den Einsatz in Flüssigkristallanzeigen sind dabei insbesondere die Anisotropien im optischen, dielektrischen und elasto-mechanischen Verhalten. Je nachdem, ob die Moleküle mit ihren Längsachsen senkrecht oder parallel zu den beiden Platten eines Kondensators orientiert sind, hat dieser eine andere Kapazität; die Dielektrizitätskonstante ε des flüssigen Kristalls ist also für die beiden Orientierungen verschieden groß. Substanzen, deren Dielektrizitätskonstante bei senkrechter Orientierung der Molekül-Längsachsen zu den Kondensatorplatten größer ist als bei paralleler Anordnung, werden als dielektrisch positiv bezeichnet. Die meisten flüssigen Kristalle, die in herkömmlichen Displays Verwendung finden, fallen in diese Gruppe.

Für die dielektrische Anisotropie spielen sowohl die Polarisierbarkeit des Moleküls als auch permanente Dipolmomente eine Rolle. Beim Anlegen einer Spannung an das Display richtet sich die Längsachse der Moleküle so aus, daß die größere der dielektrischen Konstanten wirksam wird. Die Stärke der Wechselwirkung mit dem elektrischen Feld hängt dabei von der Differenz der beiden Konstanten ab. Bei kleinen Differenzen sind höhere Schaltspannungen erforderlich als bei großen. Durch Einführen geeigneter polarer Gruppen wie Nitrile (CN-) oder Fluor in die Flüssigkristallmoleküle läßt sich ein weiter Bereich von Arbeitspannungen realisieren.

Bei den in herkömmlichen Flüssigkristallanzeigen verwendeten flüssigkristallinen Molekülen ist das entlang der Moleküllängsachse orientierte Dipolmoment größer als das senkrecht zur Moleküllängsachse orientierte Dipolmoment. Die Orientierung des größeren Dipolmoments entlang der Längsachse des Moleküls bestimmt auch die Orientierung des Moleküls in einer Flüssigkristallanzeige im feldfreien Zustand. Bei den am weitesten verbreiteten TN-Zellen (nach englisch "twisted nematic", verdrillt nematisch) ist eine nur etwa 5 bis 10 µm dicke flüssigkristalline Schicht zwischen zwei ebenen Glasplatten angeordnet, auf die jeweils eine elektrisch leitende, transparente Schicht aus Zinnoxid oder Indium-Zinnoxid als Elektrode aufgedampft ist. Zwischen diesen Filmen und der flüssigkristallinen Schicht befindet sich eine ebenfalls transparente Orientierungsschicht, die meist aus einem Kunststoff (z.B. Polyimiden) besteht. Sie dient dazu, durch Oberflächenkräfte die Längsachsen der benachbarten kristallinen Moleküle in eine Vorzugsrichtung zu bringen, so daß sie im spannungsfreien Fall einheitlich mit der gleichen Orientierung flach oder mit demselben kleinen Anstellwinkel auf der Innenseite der Displayfläche aufliegen. Auf der Außenseite des Displays sind zwei Polarisationsfolien, die nur linear polarisiertes Licht ein- und austreten lassen, in bestimmten Anordnungen aufgeklebt.

Mit Flüssigkristallen, bei denen das größere Dipolmoment parallel zur Längsachse des Moleküls orientiert ist, sind bereits sehr leistungsfähige Displays entwickelt worden. Dabei kommen meist Mischungen von 5 bis 20 Komponenten zum Einsatz, um einen ausreichend breiten Temperaturbereich der Mesophase zu erreichen sowie kurze Schaltzeiten und niedrige Schwellenspannungen. Schwierigkeiten bereitet jedoch noch die starke Blickwinkelabhängigkeit bei Flüssigkristallanzeigen, wie sie beispielsweise für Laptops verwendet werden. Die beste Abbildungsqualität läßt sich erreichen, wenn die Fläche des Displays senkrecht zur Blickrichtung des Betrachters steht. Wird das Display relativ zur Betrachtungsrichtung gekippt, verschlechtert sich die Abbildungsqualität unter Umständen drastisch. Für einen höheren Komfort ist man bemüht, den Winkel, um den das Display von der Blickrichtung eines Betrachters verkippt werden kann, möglichst groß zu gestalten. In jüngerer Zeit sind Versuche unternommen worden, zur Verbesserung der Blickwinkelabhängigkeit flüssigkristalline Verbindungen einzusetzen, deren Dipolmoment senkrecht zur Moleküllängsachse größer ist als parallel zur Längsachse des Moleküls. Im feldfreien Zustand sind diese Moleküle also senkrecht zur behandelten oder beschichteten Glasfläche des Displays orientiert. Dabei konnte eine Verbesserung der Blickwinkelabhängigkeit erreicht werden. Derartige Displays werden als VA-TFT-Displays bezeichnet von englisch "vertical align".

Die Entwicklung auf dem Gebiet der flüssigkristallinen Materialien ist bei weitem noch nicht abgeschlossen.

Optisch inaktive Benzofuran- bzw. Benzodifuranderivate, die ein- oder mehrfach fluoriert sein können, und sich als Komponenten flüssigkristalliner Medien eignen, sind aus der DE-A-19909761 bekannt.

2,3-Dihydro-6,7-difluorbenzofuran-Derivate und ihre Verwendung als Komponenten flüssigkristalliner Medien werden in der DE-A-199900517 offenbart.

In der DE-A-19840447 werden 4-Fluor- bzw. 4,5-Difluor-Derivate des Indans beschrieben, die in Flüssigkristallmischungen eingesetzt werden und bereits in geringen Mengen deren Schmelzpunkt und /oder deren dielektrische Anisotropie günstig beeinflussen.

Fluorierte Indene und 1,7-Dihydroindacene mit negativer dielektrischer Anisotropie und ihre Verwendung in flüssigkristallinen Medien sind Gegenstand der EP-A-1350780. Unter den Beispielen (Seite 16, Beispiel 2 , Verbindung 4) ist auch ein 7-Fluor-2-(4'-propyl-cyclohexyl-4-yl)-indan-1-on, welches per Disclaimer vom Schutzumfang der vorliegenden Erfindung ausgenommen wurde, da die EP-A-1350780 für dieses Beispiel eine nicht vorveröffentlichte, prioritätsältere Anmeldung gemäß Art. 54(3) und (4) EPÜ ist.

. Zur Verbesserung der Eigenschaften flüssigkristalliner Anzeigeelemente ist man ständig bemüht, neue Verbindungen zu entwickeln, die eine Optimierung derartiger Displays ermöglichen.

Aufgabe der Erfindung ist daher, flüssigkristalline Verbindungen mit vorteilhaften Eigenschaften zur Verfügung zu stellen.

Diese Aufgabe wird gelöst durch Verbindungen der Formel (Ia) oder (Ib) 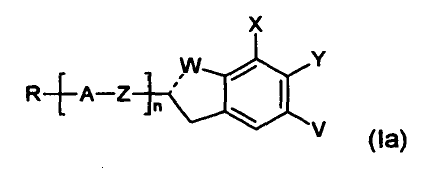 oder 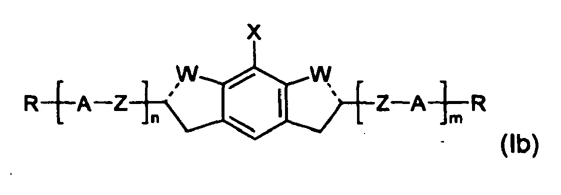 wobei bedeutet
- R: jeweils unabhängig voneinander einen unsubstituierten, einen einfach durch -CF₃ oder einen mindestens einfach durch Halogen substituierten Alkyl- oder Alkoxyrest mit 1 bis 12 C-Atomen, einen Oxaalkyl-, Alkenyl- oder Alkenyloxyrest mit 2 bis 12 C-Atomen oder einen Oxaalkenylrest mit 3 bis 12 C-Atomen, wobei in diesen Resten auch eine oder mehrere CH₂-Gruppen jeweils unabhängig voneinander durch -O-, -S-, -CO-, -COO-, -OCO- oder -OCO-O- so ersetzt sein können, daß Heteroatome nicht direkt mit einander verknüpft sind,
- A: jeweils unabhängig voneinander 1,4-Phenylen, worin =CH- ein- o-der zweimal durch =N- ersetzt sein kann, und das ein- bis viermal unabhängig voneinander mit Halogen (-F, -Cl, -Br, -I), -CN, -CH₃, -CH₂F, -CHF₂, -CF₃, -OCH₃, -OCH₂F, -OCHF₂ oder -OCF₃ substituiert sein kann, 1,4-Cyclohexylen,1,4-Cyclohexenylen oder 1,4-Cyclohexadienylen, worin -CH₂- ein- oder zweimal unabhängig voneinander durch -O- oder -S- ersetzt sein kann, und die ein- oder mehrfach durch Halogen substituiert sein können,
- Z: jeweils unabhängig voneinander eine Einfachbindung, eine -CH₂CH₂-, -CF₂CF₂- -CH=CH-, -CF=CH-, -CH=CF-, -C=C-, -CO-O-, -O-CO-, -O-CH₂-,-CH₂-O-,O-CF₂- oder eine -CF₂-O- -Gruppe,
- X: -F,
- Y, V: jeweils unabhängig voneinander Wasserstoff, einen unsubstituierten, einen einfach durch -CF₃ oder mindestens einfach durch Halogen substituierten Alkylrest, Alkoxyrest, Alkenylrest oder Alkinylrest mit 1 bis 15 bzw. 2 bis 15 C-Atomen, wobei in diesen Resten auch eine oder mehrere CH₂-Gruppen durch -O-, -S-, -COO-, -CO-, -OCO- oder -OCO-O- so ersetzt sein können, dass Hetero-atome nicht direkt benachbart sind,
- Y: zusätzlich auch -F oder -Cl,

- W: jeweils unabhängig voneinander -O-, -C(O)-, -CHF- oder -CF₂- bzw. -CF= und zusätzlich auch -CH₂- in Formel (lb),
- n, m: jeweils unabhängig voneinander 1 oder 2,
und die gepunktete Linie für eine Einfach- oder eine Doppelbindung steht, mit der Maßgabe, dass die Verbindung der Formel (Ia), worin R = Propyl, A = 1,4-Cyclohexylen, Z = eine Einfachbindung, X = -F, Y und V = Wasserstoff, W = -C(O)- und n = 1 ausgenommen ist. In den allgemeinen Formeln (Ia) und (Ib) sind A jeweils unabhängig voneinander bevorzugt gegebenenfalls substituiertes 1,4-Phenylen, gegebenenfalls substituiertes 1,4-Cyclohexylen, worin -CH₂- ein- oder zweimal durch -O- ersetzt sein kann, oder gegebenenfalls substituiertes 1,4-Cyclohexenylen.

Besonders bevorzugt sind A jeweils unabhängig voneinander

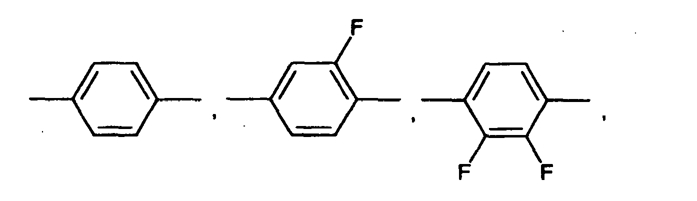

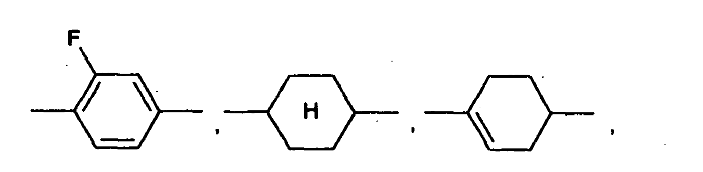

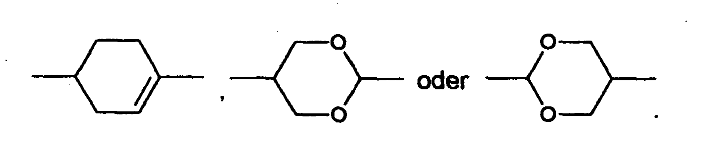

R, Y und V in den allgemeinen Formeln (la) und (Ib) können jeweils unabhängig voneinander ein Alkylrest und/oder einen Alkoxyrest mit 1 bis 15 C-Atomen sein, der geradkettig oder verzweigt sein kann. Vorzugsweise ist er geradkettig, hat 1, 2, 3, 4, 5, 6 oder 7 C-Atome und ist demnach bevorzugt Methyl, Ethyl, Propyl, Butyl, Pentyl, Hexyl, Heptyl, Methoxy, Ethoxy, Propoxy, Butoxy, Pentoxy, Hexoxy oder Heptoxy.
R, Y und V können jeweils unabhängig voneinander Oxaalkyl sein, vorzugweise geradkettiges 2-Oxapropyl (= Methoxymethyl), 2- (= Ethoxymethyl) oder 3-Oxabutyl (= Methoxyethyl), 2-, 3- oder 4-Oxapentyl, 2-, 3-, 4- oder 5-Oxahexyl, 2-, 3-, 4-, 5- oder 6-Oxaheptyl.

R, Y und W können jeweils unabhängig voneinander ein Alkenylrest mit 2 -15 C-Atomen sein, der geradkettig oder verzweigt sein kann. Vorzugsweise ist er geradkettig und hat 2 bis 7 C-Atome. Er ist demnach insbesondere Vinyl, Prop-1-, oder Prop-2-enyl, But-1-, 2- oder But-3-enyl, Pent-1-,2-,3- oder Pent-4-enyl, Hex-1-, 2-, 3-, 4- oder Hex-5-enyl, Hept-1-, 2-, 3-, 4-, 5- oder Hept-6-enyl.

R, Y, und V können jeweils unabhängig voneinander ein Alkylrest mit 1 bis 15 C-Atomen sein, in dem eine CH₂-Gruppe durch -0- und eine durch -CO- ersetzt ist, wobei diese bevorzugt benachbart sind. Somit beinhaltet dieser eine Acyloxygruppe -CO-O- oder eine Oxycarbonylgruppe -O-CO-. Vorzugsweise ist dieser geradkettig und hat 2 bis 6 C-Atome.

R, Y und V können jeweils unabhängig voneinander ein Alkylrest mit 1 bis 15 C-Atomen sein, in dem eine CH₂-Gruppe durch unsubstituiertes oder substituiertes -CH=CH- und eine benachbarte CH₂-Gruppe durch -CO- oder -CO-O- oder -O-CO- ersetzt ist, wobei dieser geradkettig oder verzweigt sein kann. Vorzugsweise ist er geradkettig und hat 4 bis 13 C-Atome.

R, Y und V können jeweils unabhängig voneinander ein einfach durch -CF₃ substituierter Alkylrest mit 1 bis 15 C-Atomen oder Alkenylrest mit 2 bis 15 C-Atomen sein, wobei diese vorzugsweise geradkettig sind. Die Substitution durch -CF₃ ist in beliebiger Position.

R, Y und V können jeweils unabhängig voneinander ein mindestens einfach durch Halogen substituierter Alkylrest mit 1 bis 15 C-Atomen oder Alkenylrest mit 2 bis 15 C-Atomen sein, wobei diese Reste vorzugsweise geradkettig sind und Halogen vorzugsweise -F oder -Cl ist. Bei Mehrfach-substitution ist Halogen vorzugsweise -F. Die resultierenden Reste schließen auch perfluorierte Rest ein. Bei Einfachsubstitution kann der Fluor- oder Chlorsubstituent in beliebiger Position sein, vorzugsweise ist er in ω-Position.

R, Y und V können jeweils unabhängig voneinander ein Alkylrest sein; in dem zwei oder mehr CH₂-Gruppen durch -O- und/oder -CO-O- ersetzt sind, wobei dieser geradkettig oder verzweigt sein kann. Vorzugsweise ist er verzweigt und hat 3 bis 12 C-Atome.

Bevorzugt sind R, Y und V in den allgemeinen Formeln (la) und (Ib) Wasserstoff oder ein Alkylrest, Alkoxyrest oder Alkenylrest mit 1 bis 7 bzw. 2 bis 7 C-Atomen.

Y ist zusätzlich bevorzugt -F oder -Cl, insbesondere -F.

Bevorzugte Indane der allgemeinen Formel (Ia), bzw. (Ib) weisen einen oder zwei Cyclen A auf.

Die Verbindungen besitzen ein negatives Δε und eignen sich daher für eine Verwendung in VA-TFT-Displays. Sie zeigen eine sehr gute Verträglichkeit mit den üblichen, in Flüssigkristallmischungen für Displays verwendeten Substanzen.

Durch die Substituenten X und W im Indangerüst wird ein Dipolmoment senkrecht zur Moleküllängsachse erzeugt, das gegebenenfalls durch geeignete Substituenten in den Flügeleinheiten ZAZAR weiter verstärkt werden kann. Im feldfreien Zustand richten sich die Verbindungen der Formeln (la) bzw. (Ib) mit ihrer Moleküllängsachse senkrecht zur Glasfläche eines Displays aus.

Als besonders geeignet haben sich die im folgenden aufgeführten Gruppen von Verbindungen gezeigt, wobei W, V, A, Y, Z, R, n und m die oben angegebene Bedeutung aufweisen. 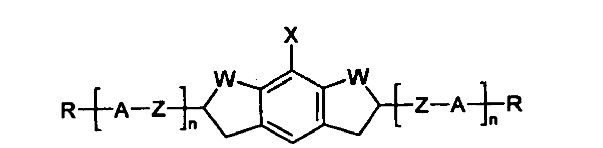 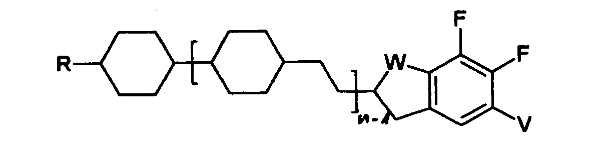 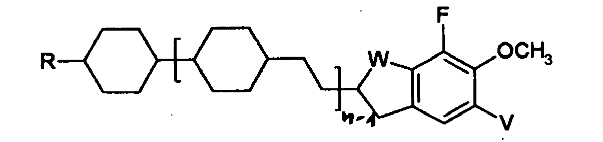 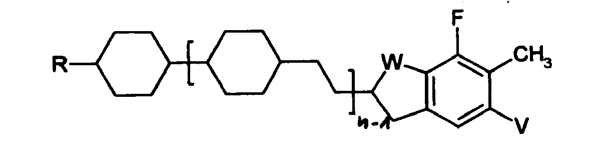 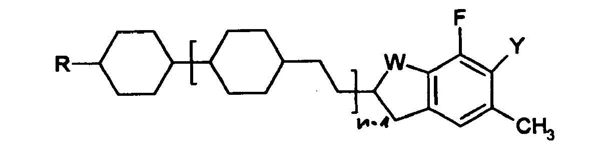 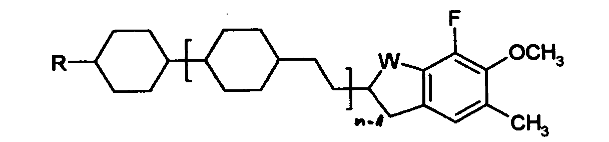 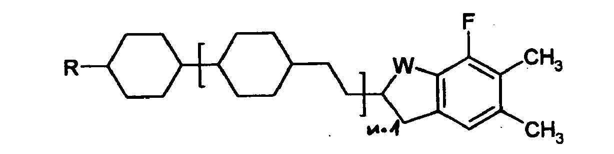 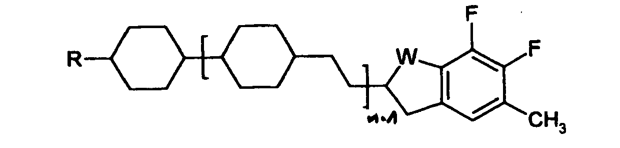 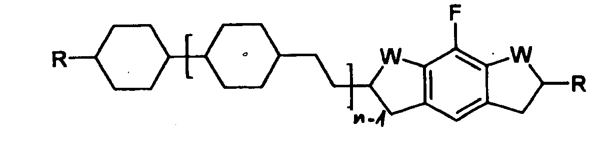 Insbesondere geeignet sind die folgenden Verbindungen: 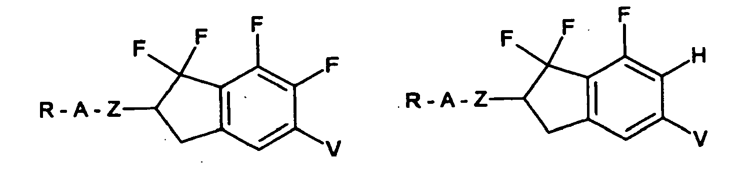 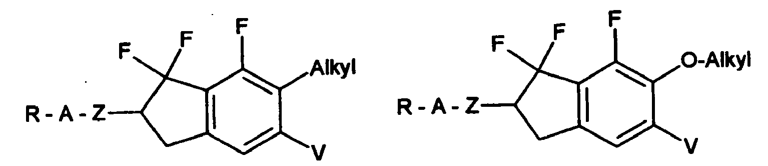 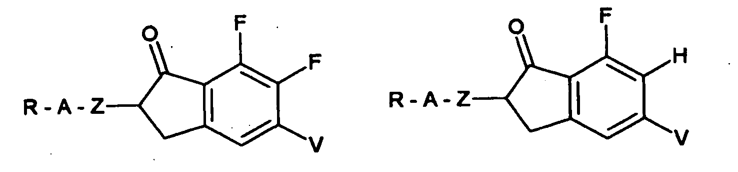 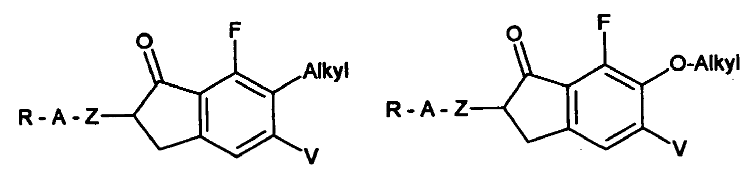 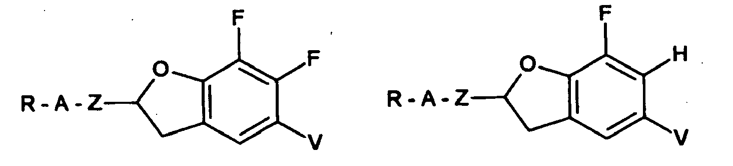 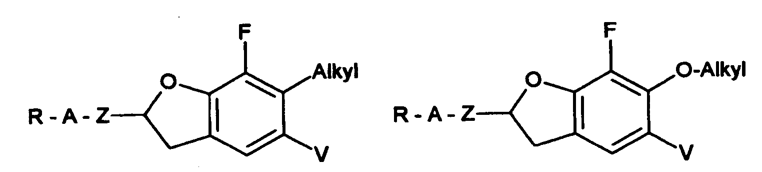 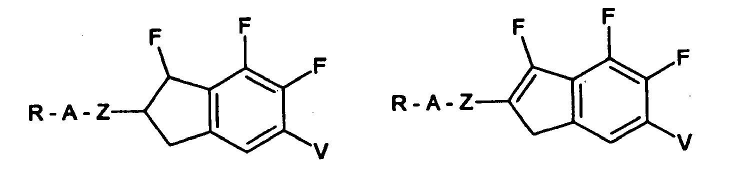 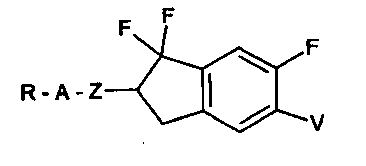 mit

| R | A | Z | V |
|---|---|---|---|
| n-Alkyl | 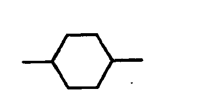 | --- | H |
| n-Alkyl | 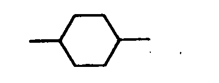 | --- | n-Alkyl |
| n-Alkyl | 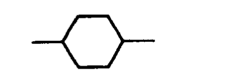 | --- | -O-n-Alkyl |
| n-Alkyl | 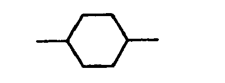 | -CH₂-CH₂ | H |
| n-Alkyl | 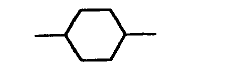 | -CH₂-CH₂- | n-Alkyl |
| n-Alkyl | 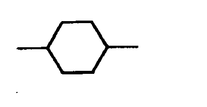 | -CH₂-CH₂ | -O-n-Alkyl |
| n-Alkyl | 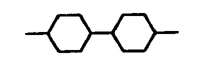 | --- | H |
| n-Alkyl | 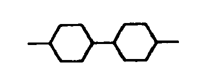 | --- | n-Alkyl |
| n-Alkyl | 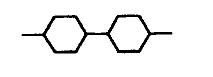 | --- | -O-n-Alkyl |
| n-Alkyl | 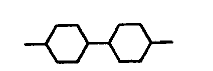 | -CH₂-CH₂- | H |
| n-Alkyl | 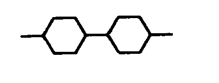 | -CH₂-CH₂- | n-Alkyl |
| n-Alkyl | 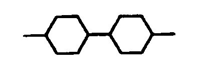 | -CH₂-CH₂- | -O-n-Alkyl |
| n-Alkyl | 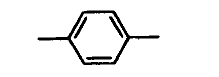 | --- | H |
| n-Alkyl | 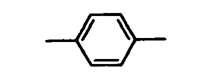 | --- | n-Alkyl |
| n-Alkyl | 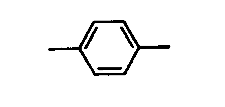 | --- | -O-n-Alkyl |
| n-Alkyl | 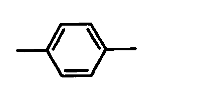 | -CH₂-CH₂- | H |
| n-Alkyl | 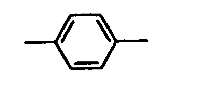 | -CH₂-CH₂- | n-Alkyl |
| n-Alkyl | 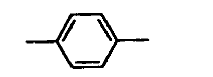 | -CH₂-CH₂- | -O-n-Alkyl |
| n-Alkyl | 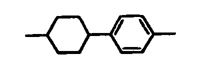 | --- | H |
| n-Alkyl | 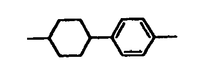 | --- | n-Alkyl |
| n-Alkyl | 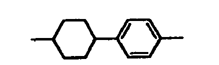 | --- | -O-n-Alkyl |
| n-Alkyl | 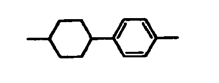 | -CH₂-CH₂- | H |
| n-Alkyl | 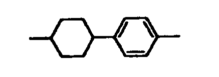 | -CH₂-CH₂- | n-Alkyl |
| n-Alkyl | 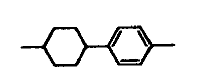 | -CH₂-CH₂- | -O-n-Alkyl |
| n-Alkyl | 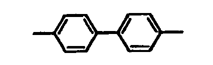 | --- | H |
| n-Alkyl | 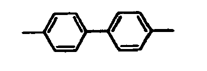 | --- | n-Alkyl |
| n-Alkyl | 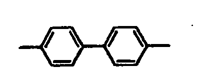 | --- | -O-n-Alkyl |
| | 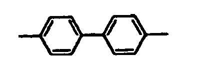 | -CH₂-CH₂- | H |
| n-Alkyl | 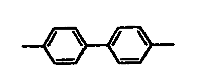 | -CH₂-CH₂- | n-Alkyl |
| n-Alkyl | 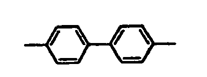 | -CH₂-CH₂- | -O-n-Alkyl |
| n-Alkyl | 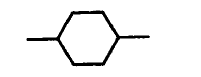 | -CH₂-O- | H |
| n-Alkyl | 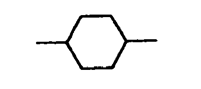 | -CH₂-O- | n-Alkyl |
| n-Alkyl | 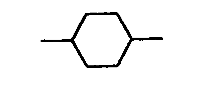 | -CH₂-O- | -O-n-Alkyl |
| n-Alkyl | 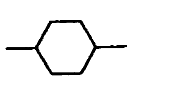 | -O-CH₂ | H |
| n-Alkyl | 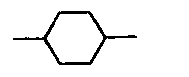 | -O-CH₂ | n-Alkyl |
| n-Alkyl | 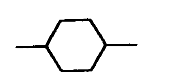 | -O-CH₂- | -O-n-Alkyl |
| n-Alkyl | 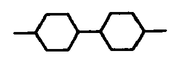 | -CH₂-O- | H |
| n-Alkyl | 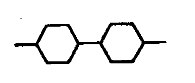 | -CH₂-O- | n-Alkyl |
| n-Alkyl | 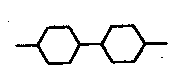 | -CH₂-O- | -O-n-Alkyl |
| n-Alkyl | 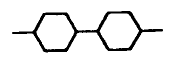 | -O-CH₂- | H |
| n-Alkyl | 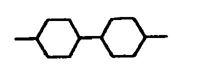 | -O-CH₂- | n-Alkyl |
| n-Alkyl | 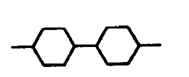 | -O-CH₂- | -O-n-Alkyl |
| n-Alkyl | 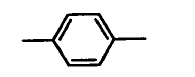 | -O-CH₂- | H |
| n-Alkyl | 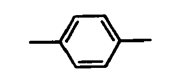 | -O-CH₂- | n-Alkyl |
| n-Alkyl | 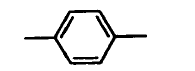 | -O-CH₂- | -O-n-Alkyl |
| n-Alkyl | 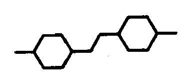 | --- | H |
| n-Alkyl | 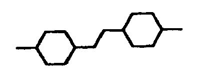 | --- | n-Alkyl |
| n-Alkyl | 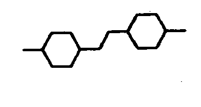 | --- | -O-n-Alkyl |
| n-Alkyl | 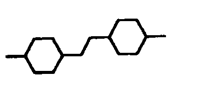 | -CH₂-CH₂- | H |
| n-Alkyl | 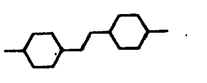 | -CH₂-CH₂- | n-Alkyl |
| n-Alkyl | 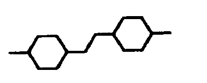 | -CH₂-CH₂- | -O-n-Alkyl |
| n-Alkyl | 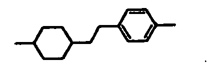 | --- | H |
| n-Alkyl | 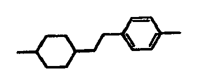 | --- | n-Alkyl |
| n-Alkyl | 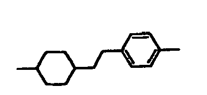 | --- | -O-n-Alkyl |
| n-Alkyl | 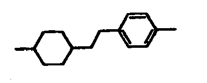 | -CH₂-CH₂- | H |
| n-Alkyl | 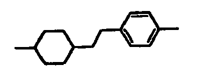 | -CH₂-CH₂- | n-Alkyl |
| n-Alkyl | 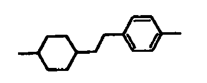 | -CH₂-CH₂- | -O-n-Alkyl |
| n-Alkyl-O- | 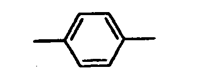 | --- | H |
| n-Alkyl-O- | 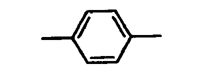 | --- | n-Alkyl |
| n-Alkyl-O- | 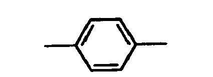 | --- | -O-n-Alkyl |
| n-Alkyl | 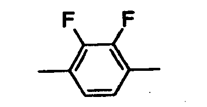 | --- | H |
| n-Alkyl | 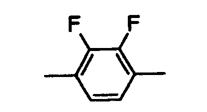 | --- | n-Alkyl |
| n-Alkyl | 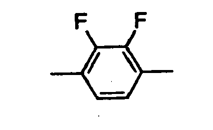 | --- | -O-n-Alkyl |
| n-Alkyl | 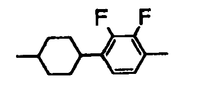 | --- | H |
| n-Alkyl | 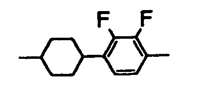 | --- | n-Alkyl |
| n-Alkyl | 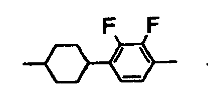 | --- | -O-n-Alkyl |
| n-Alkyl | 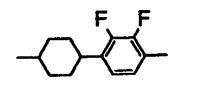 | -CH₂-CH₂- | H |
| n-Alkyl | 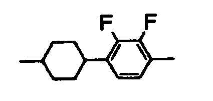 | -CH₂CH₂- | n-Alkyl |
| n-Alkyl | 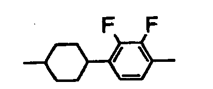 | -CH₂-CH₂- | -O-n-Alkyl |
| n-Alkyl | 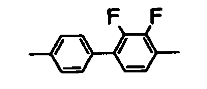 | --- | H |
| n-Alkyl | 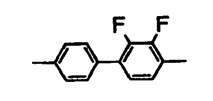 | --- | n-Alkyl |
| n-Alkyl | 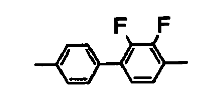 | --- | -O-n-Alkyl |
| n-Alkyl-O- | 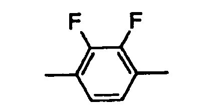 | --- | H |
| n-Alkyl-O- | 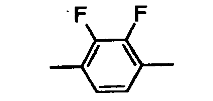 | --- | n-Alkyl |
| n-Alkyl-O- | 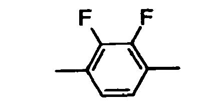 | --- | -O-n-Alkyl |

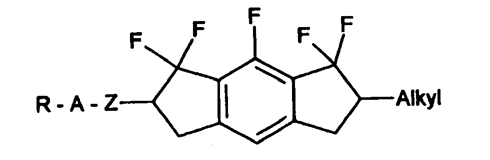

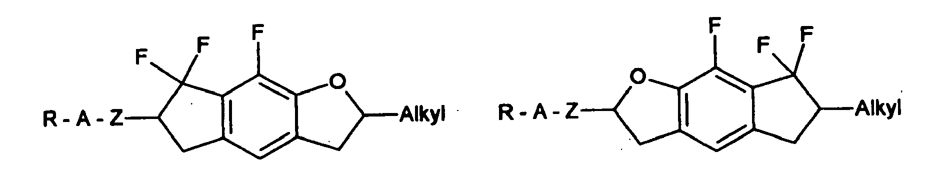

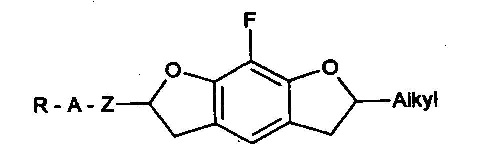

mit

| R | A | Z |
|---|---|---|
| n-Alkyl | 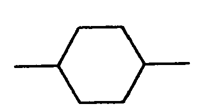 | --- |
| n-Alkyl | 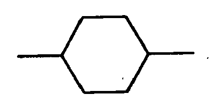 | -CH₂-CH₂- |
| n-Alkyl | 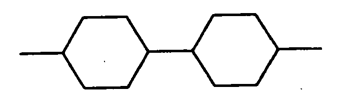 | --- |
| n-Alkyl | 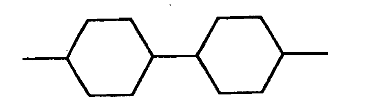 | -CH₂-CH₂- |
| n-Alkyl | 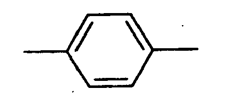 | --- |
| n-Alkyl | 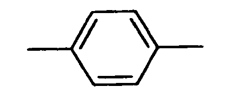 | -CH₂CH₂- |
| n-Alkyl | 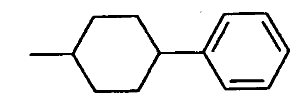 | --- |
| n-Alkyl | 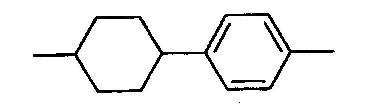 | -CH₂-CH₂- |
| n-Alkyl | 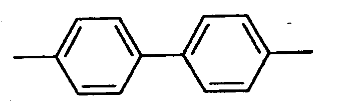 | --- |
| n-Alkyl | 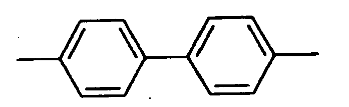 | -CH₂-CH₂- |
| n-Alkyl | 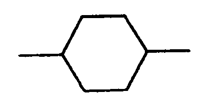 | -CH₂-O- |
| n-Alkyl | 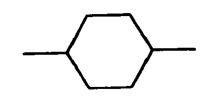 | -O-CH₂- |
| n-Alkyl | 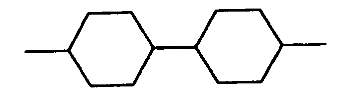 | -CH₂-O- |
| n-Alkyl | 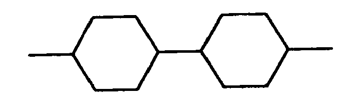 | -O-CH₂- |
| n-Alkyl | 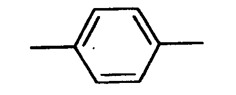 | -O-CH₂- |
| n-Alkyl | 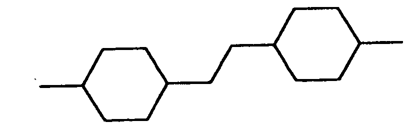 | --- |
| n-Alkyl | 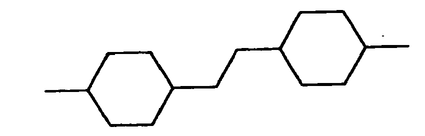 | -CH₂-CH₂- |
| n-Alkyl | 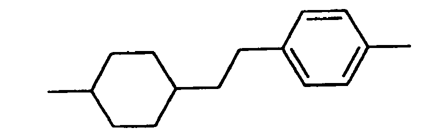 | --- |
| n-Alkyl | 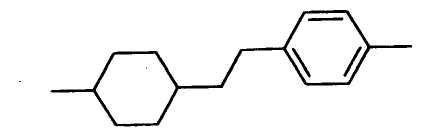 | -CH₂-CH₂- |
| n-alkyl-O | 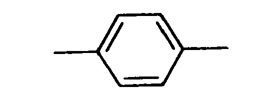 | --- |
| n-Alkyl | 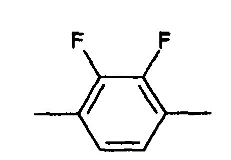 | --- |
| n-Alkyl | 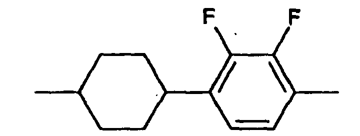 | --- |
| n-Alkyl | 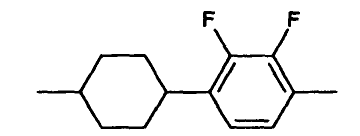 | -CH₂-CH₂- |
| n-Alkyl | 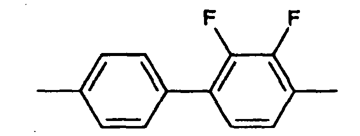 | --- |
| n-Alkyl | 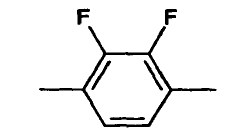 | --- |

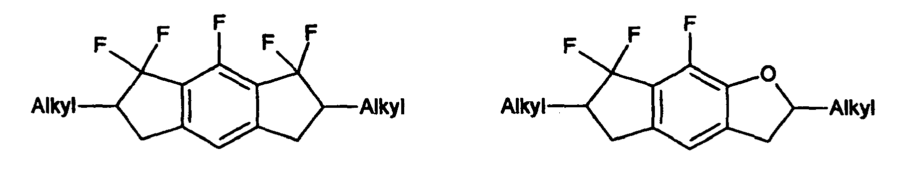

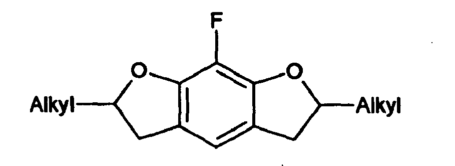

Ganz besonders bevorzugt sind die folgenden Strukturen:

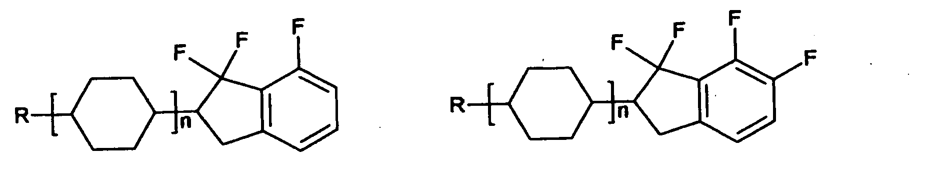

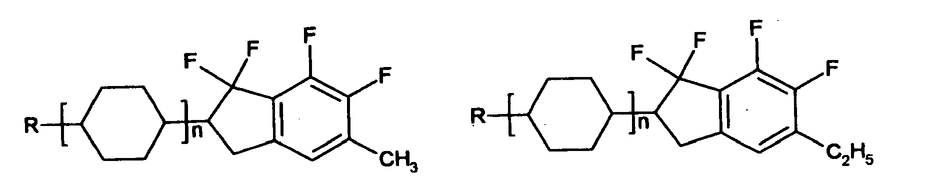

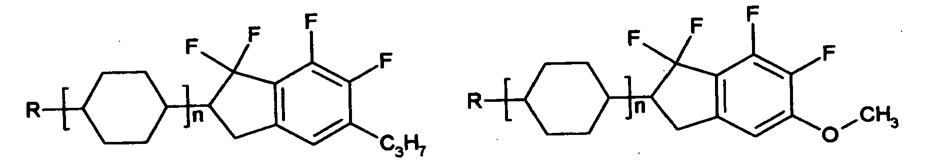

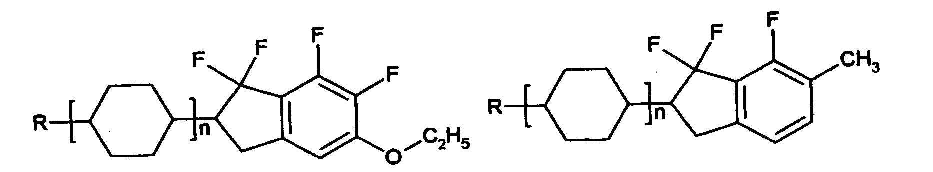

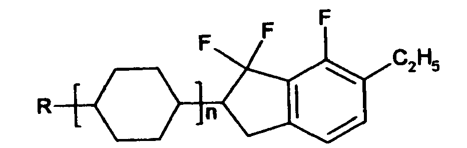

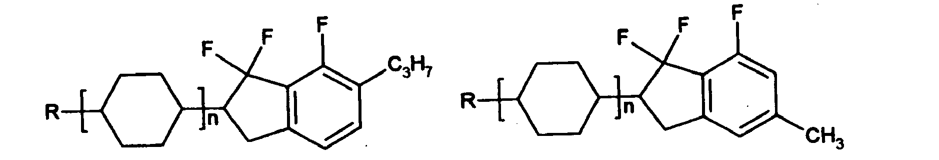

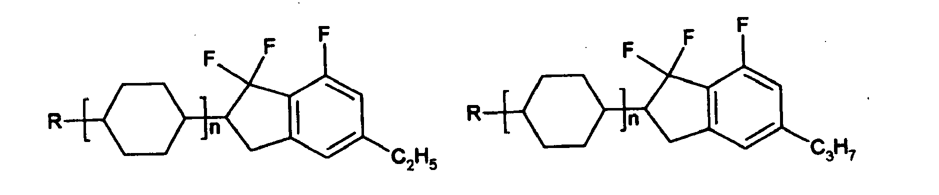

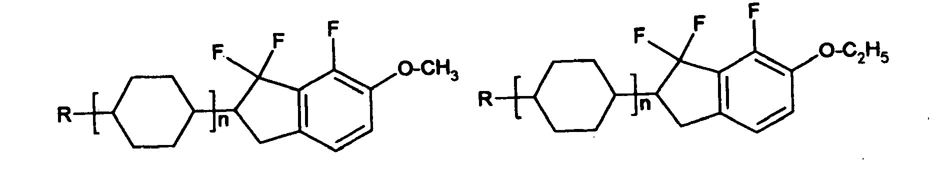

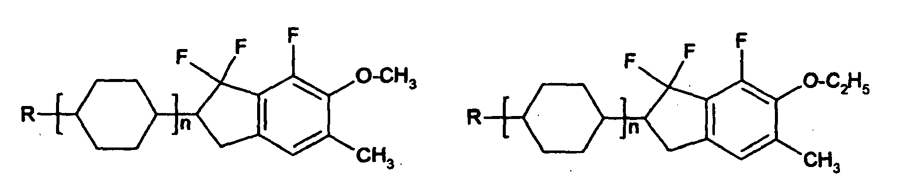

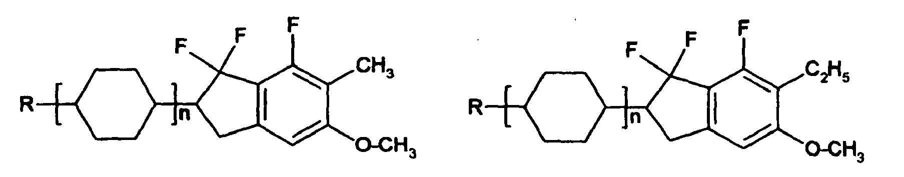

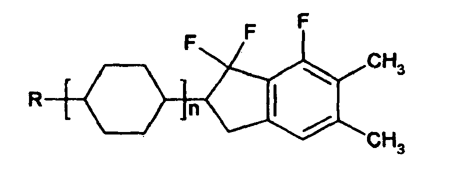

Unter den zuletzt als ganz besonders bevorzugt genannten Strukturen sind diejenigen insbesondere bevorzugt, in denen
- n =1: und R = n-Alkyl, insbesondere C₁₋C₅-Alkyl,
- n = 2: und R = n-Alkyl, insbesondere C₁-C₅-Alkyl,

- n = 1: und R = n-Alkenyl, insbesondere Vinyl, Prop-1-enyl, But-1-enyl und But-3-enyl,
- n = 2: und R = n-Alkenyl, insbesondere Vinyl, Prop-1-enyl, But-1-enyl und But-3-enyl
ist.

Die Verbindungen der Formel (la) und (Ib) werden nach an sich bekannten Methoden dargestellt, wie sie in der Literatur (z.B. in den Standardwerken wie Houben-Weyl, Methoden der organischen Chemie, Georg-Thieme-Verlag, Stuttgart) beschrieben sind und zwar unter Reaktionsbedingungen, die für die genannten Umsetzungen bekannt und geeignet sind. Dabei kann man von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel (I) bzw. (Ib) umsetzt.

Eine beispielhafte Synthese ist im folgenden dargestellt. Die Synthese kann durch die Wahl geeigneter Ausgangsprodukte an die jeweils gewünschten Verbindungen der Formel (I) bzw. (Ib) angepaßt werden.

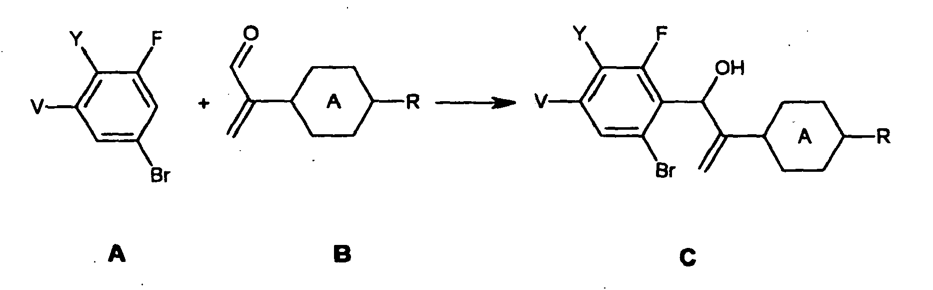

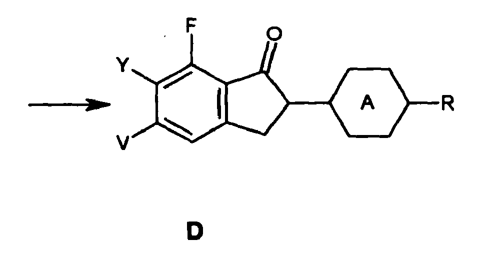

Ausgehend vom 3-Bromfluorbenzol A wird durch Umsetzung mit dem α, βungesättigten Aldehyd B in Gegenwart von Lithiumdiisopropylamid die Verbindung C erhalten. Diese reagiert unter Palladiumkatalyse in Gegenwart von Triethylamin unter Ringschluss zum Indanon D.

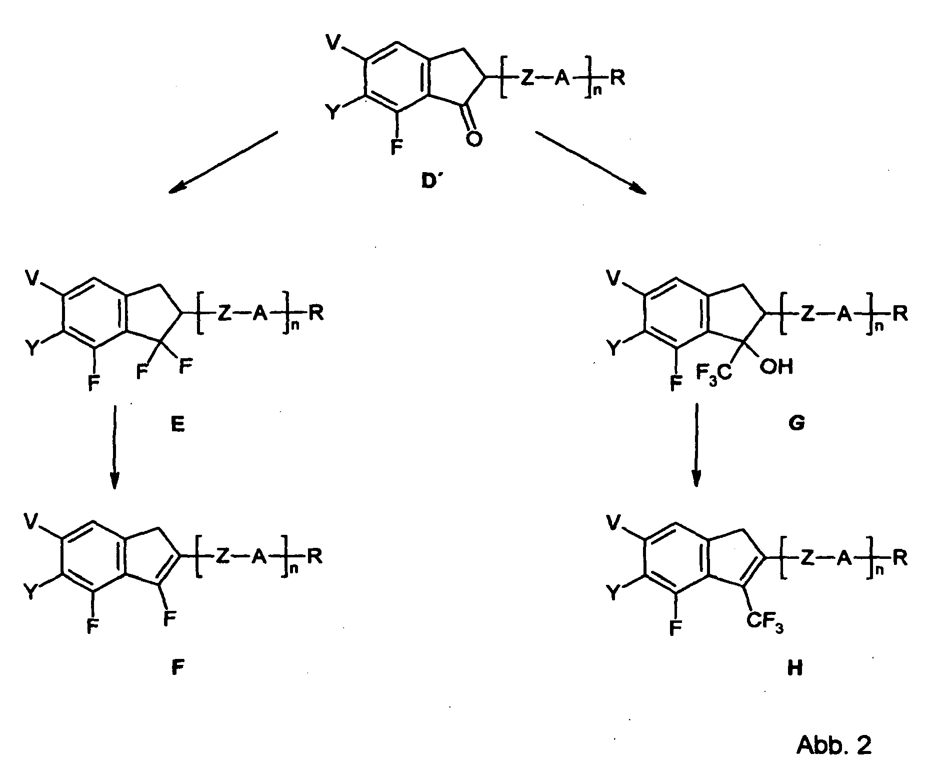

Ausgehend von der Ketoverbindung **D**' wird durch Fluorierung mit einem geeigneten Fluorierungsmittel, wie DAST oder SF₄, die Difluorverbindung **E** erhalten, aus der dann gegebenenfalls mit einer starken Base wie Kalium-tert.-butylat Fluorwasserstoff abgespalten werden kann zur Darstellung der Verbindung **F**. Durch Umsetzung mit F₃CSi(CH₃)₃ und anschließender Behandlung mit KF/CH₃OH kann eine Trifluormethylgruppe in das Molekül eingeführt werden (**G**). Anschließend wird mit SOCl₂/Pyridin zur Verbindung **H** dehydratisiert.

Das Indanon **D**' kann auch gemäß Abb. 2a weiter umgesetzt werden.

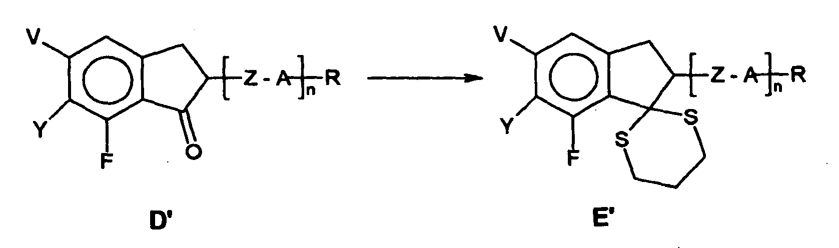

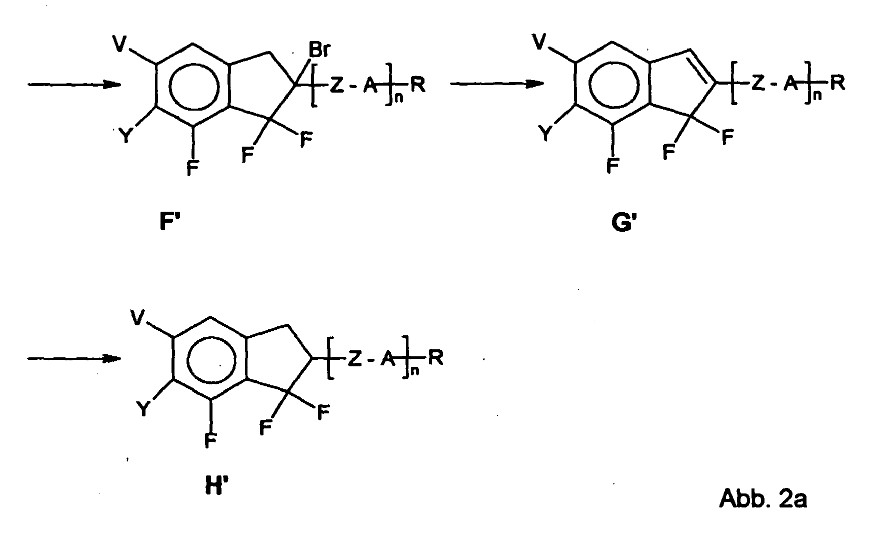

Die Ketoverbindung D' wird mit Propan-1,3-dithiol zum cyclischen Thioketal E' umgesetzt. Aus diesem wird mit HF-Pyridin in Gegenwart von Dibromdimethylhydantoin bereits das Endprodukt H' erhalten, in gewissen Anteilen aber auch das Bromid F'. Das Gemisch wird mit Base behandelt, um aus der Verbindung F' HBr abzuspalten, wodurch die Verbindung G' erhalten wird. Die Verbindung G' wird anschließend in dem Gemisch aus G' und H' zum Endprodukt H' hydriert.

Weitere Möglichkeiten zur Ausbildung des Indangerüstes sind in Abb. 3 dargestellt.

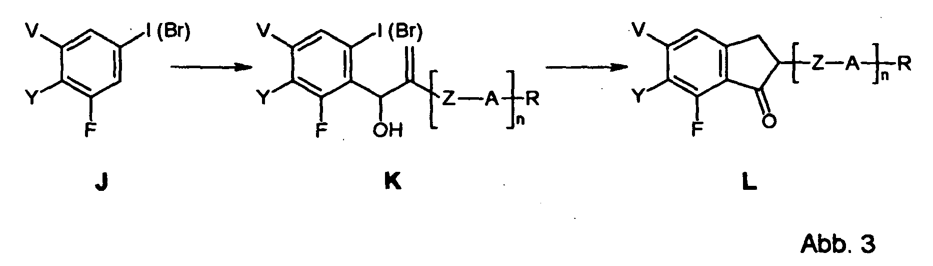

Ausgehend von dem geeignet substituierten lodbenzolderivat J wird zunächst bei tiefer Temperatur mit einer starken Base, wie LDA, deprotoniert und anschließend mit einem geeigneten Acroleinderivat zum Alkohol K umgesetzt. Der Ringschluß zur Verbindung L erfolgt anschließend durch Heck-Reaktion.

Erfindungsgemäße Dihydrobenzofurane können gemäß der in Abb. 4 dargestellten allgemeinen Reaktionsfolge dargestellt werden.

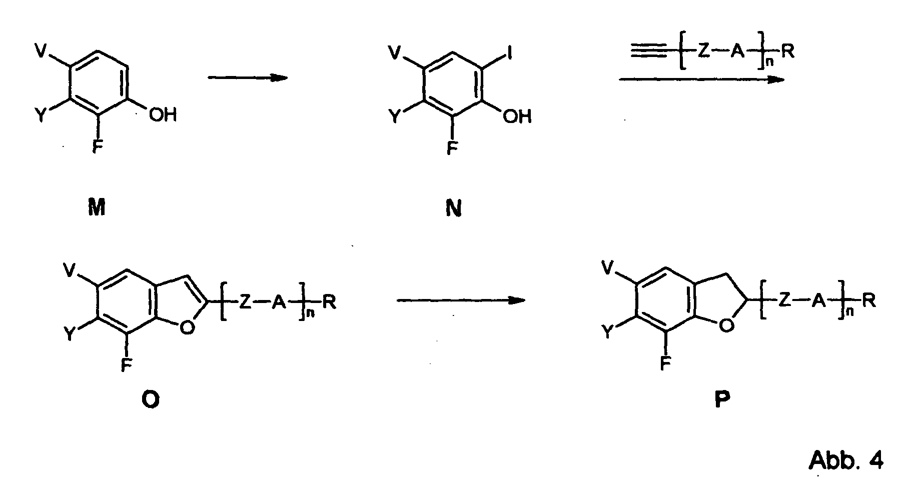

Ausgehend vom Phenolderivat M wird zunächst durch Umsetzung mit lod unter Wirkung einer schwachen Base eine lodgruppe unter Erhalt der Verbindung N eingeführt. Anschließend erfolgt Ringschluß durch Umsetzung mit einem geeigneten Acetylenderivat unter Katalyse durch eine Palladium(II)verbindung, wie Palladium(II)acetat zum Benzofuranderivat O. Dieses kann dann durch katalytische Hydrierung an Palladium/Kohle zum Dihydrobenzofuranderivat P hydriert werden.

Erfindungsgemäße Indacene können gemäß der in Abb. 5 dargestellten Reaktionsfolge erhalten werden.

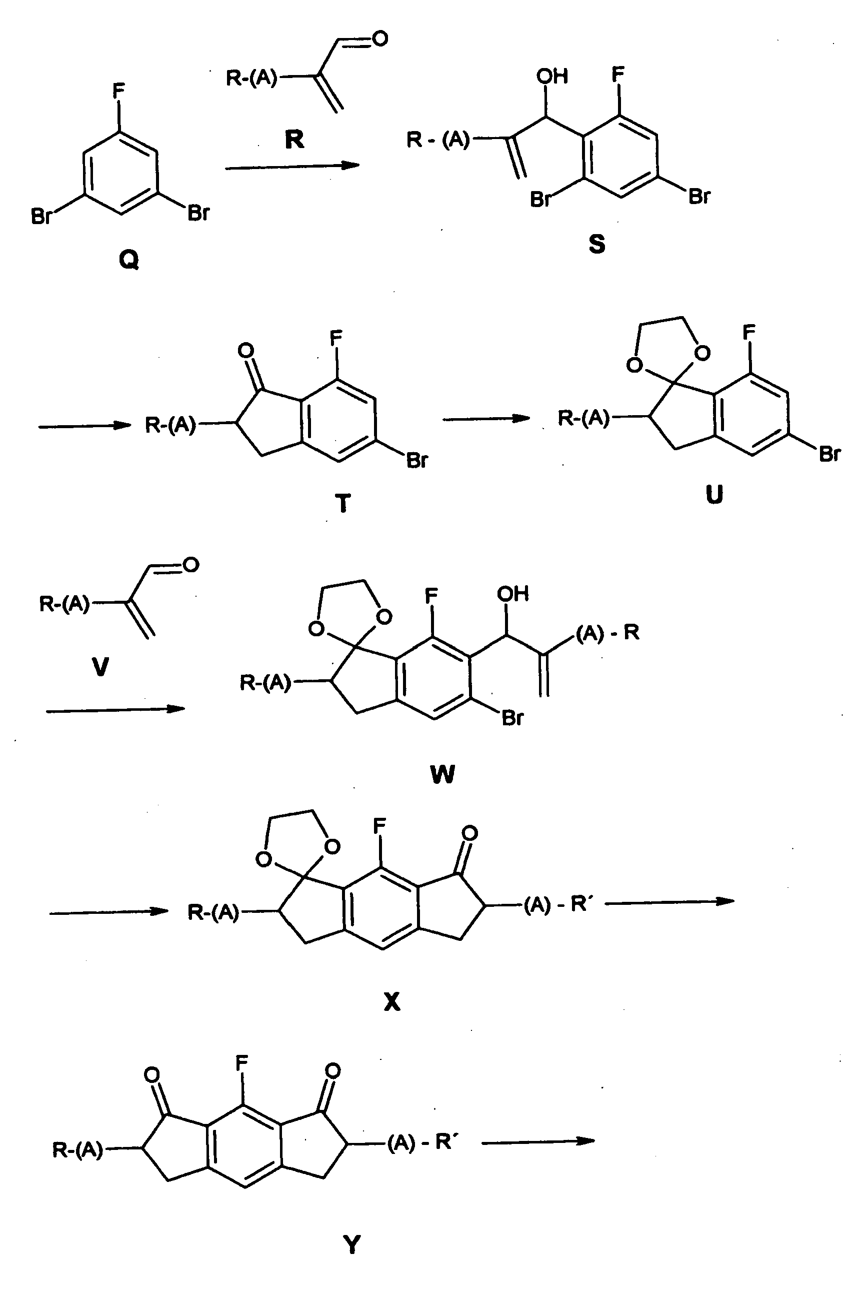

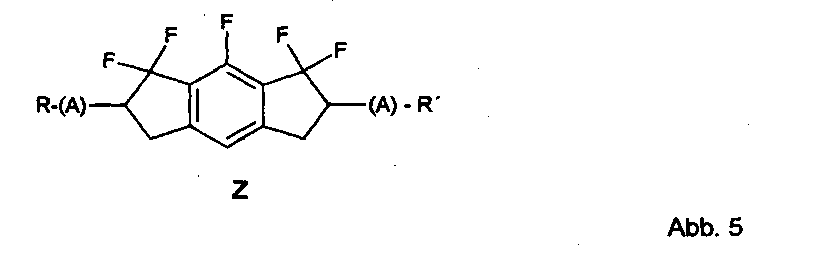

Ausgehend von 3,5-Dibromfluorbenzol Q wird durch Umsetzung mit dem ungesättigten Aldehyd R in Gegenwart von Lithiumdiisopropylamid die Verbindung S erhalten. Diese reagiert unter Palladiumkatalyse in Gegenwart von Triethylamin unter Ringschluß zum Indanon T. Nach Ketalisierung der Ketogruppe mit Ethylenglycol in Gegenwart von Toluolsulfonsäure wird dieses Verfahren mit dem ungesättigten Aldehyd V wiederholt und so Indacen X erhalten. Nach dem Entfernen der Schutz- gruppe mit Hilfe einer Säure erhält man das Diketon Y , welches sich mit Hilfe geeigneter Fluorierungsmittel wie SF₄ zum Endprodukt Z überführen läßt.

Die dargestellten Reaktionen sind nur als beispielhaft aufzufassen. Der Fachmann kann entsprechende Variationen der vorgestellten Synthesen vornehmen sowie auch andere geeignete Synthesewege beschreiten, um Verbindungen der Formeln (la) und (Ib) zu erhalten.

Wie bereits erwähnt, können die Verbindungen der Formeln (la) und (Ib) zur Herstellung flüssigkristalliner Mischungen verwendet werden. Gegenstand der Erfindung ist daher auch ein flüssigkristallines Medium mit mindestens zwei flüssigkristallinen Verbindungen, umfassend mindestens eine Verbindung der Formeln (la) und (Ib).

Gegenstand der vorliegenden Erfindung sind auch flüssigkristalline Medien enthaltend neben einer oder mehreren erfindungsgemäßen Verbindungen der Formeln (la) und/oder (Ib) als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexancarbonsäurephenyl- oder-cyclohexylester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Biscyclohexylbenzole, 4',4'-Biscyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenylcyclohexyl)-ethane, 1-Cyclohexyl-2-biphenylylethane, 1-Phenyl-2-cyclohexylphenylethane, gegebenenfalls halogenierten Stilbene, Benzylphenylether, Tolane und substituierten Zimtsäuren. Die 1,4-Phenylen-gruppen in diesen Verbindungen können auch fluoriert sein.

Die wichtigsten als weitere Bestandteile erfindungsgemäßer Medien in Frage kommenden Verbindungen lassen sich durch die Formeln (II), (III), (IV), (V) und (VI) charakterisieren:

R'-L-E-R" (II)

R'-L-COO-E-R" (III)

R'-L-OOC-E-R" (IV)

R'-L-CH₂CH₂-E-R" (V)

R'-L-CF₂O-E-R" (VI)

In den Formeln (II), (III), (IV), (V) und (VI) bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc oder Phe. E ist vorzugsweise Cyc, Phe oder Phe-Cyc. Vorzugsweise enthalten die erfindungsgemäßen Medien eine oder mehrere Komponenten ausgewählt aus den Verbindungen der Formeln (II), (III), (IV), (V) und (VI), worin L und E ausgewählt sind aus der Gruppe Cyc und Phe und gleichzeitig eine oder mehrere Komponenten ausgewählt aus den Verbindungen der Formeln (II), (III), (IV), (V) und (VI), worin einer der Reste L und E ausgewählt ist aus der Gruppe Cyc und Phe und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln (II), (III), (IV), (V) und (VI), worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln (II), (III), (IV), (V) und (VI) jeweils unabhängig voneinander Alkyl, Alkenyl, Alkoxy, Alkoxyalkyl, Alkenyloxy oder Alkanoyloxy mit bis zu 8 Kohlenstoffatomen. Im Folgenden wird diese kleinere Untergruppe Gruppe A genannt und die Verbindungen werden mit den Teilformeln (IIa), (IIIa), (IVa), (Va) und (VIa) bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln (II), (III), (IV), (V) und (VI) bedeutet E 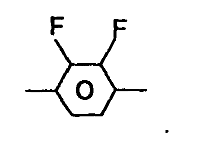 In den Verbindungen der Gruppe B, die mit den Teilformeln (IIb), (IIIb), (IVb), (Vb) und (VIb) bezeichnet werden, haben R' und R" die bei den Verbindungen der Teilformeln (IIa) bis (VIa) angegebene Bedeutung und sind vorzugsweise Alkyl, Alkenyl, Alkoxy oder Alkoxyalkyl.

In einer weiteren kleineren Untergruppe der Verbindungen der Formeln (II), (III), (IV), (V) und (VI) bedeutet R" -CN; diese Untergruppe wird im Folgenden als Gruppe C bezeichnet und die Verbindungen dieser Untergruppe werden entsprechend mit Teilformeln (IIc), (IIIc), (IVc), (Vc) und (VIc) beschrieben. In den Verbindungen der +Teilformeln (IIc), (IIIc), (IVc), (Vc) und (VIc) hat R' die bei den Verbindungen der Teilformeln (IIa), (IIIa), (I-Va), (Va) und (VIa) angegebene Bedeutung und ist vorzugsweise Alkyl, Alkoxy oder Alkenyl.

Neben den bevorzugten Verbindungen der Gruppen A, B und C sind auch andere Verbindungen der Formeln (II), (III), (IV), (V) und (VI) mit anderen Varianten der vorgesehenen Substituenten gebräuchlich. All diese Substanzen sind nach literaturbekannten Methoden oder in Analogie dazu erhältlich.

Die erfindungsgemäßen Medien enthalten neben erfindungsgemäßen Verbindungen der Formeln (la) und/oder (Ib) vorzugsweise eine oder mehrere Verbindungen, welche ausgewählt werden aus der Gruppe A und/oder Gruppe B und/oder Gruppe C. Die Massenanteile der Verbindungen aus diesen Gruppen an den erfindungsgemäßen Medien sind vorzugsweise

Gruppe A: 0 bis 90%, vorzugsweise 20 bis 90%, insbesondere 30 bis 90% Gruppe B: 0 bis 80%, vorzugsweise 10 bis 80%, insbesondere 10 bis 70% Gruppe C: 0 bis 80%, vorzugsweise 5 bis 80%, insbesondere 5 bis 50%

wobei die Summe der Massenanteile der in den jeweiligen erfindungsgemäßen Medien enthaltenen Verbindungen aus den Gruppen A und/oder B und/oder C vorzugsweise 5 bis 90% und insbesondere 10 bis 90% beträgt.

Die erfindungsgemäßen Medien enthalten vorzugsweise 1 bis 40%, insbesondere vorzugsweise 5 bis 30% an erfindungsgemäßen Verbindungen der Formeln (la) und/oder (Ib). Weiterhin bevorzugt sind Medien, enthaltend mehr als 40%, insbesondere 45 bis 90% an erfindungsgemäßen Verbindungen der Formeln (Ia) und/oder (Ib). Die Medien enthalten vorzugsweise drei, vier oder fünf erfindungsgemäße Verbindungen der Formeln (Ia) und/oder (Ib).

Beispiele für die Verbindungen der Formeln (II), (III), (IV), (V) und (VI) sind die nachstehend aufgeführten Verbindungen: 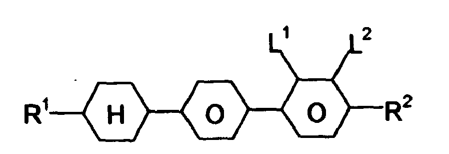 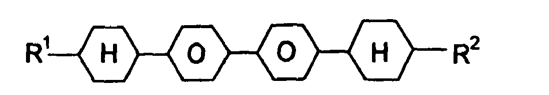 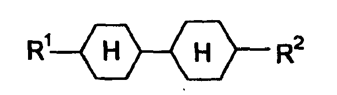 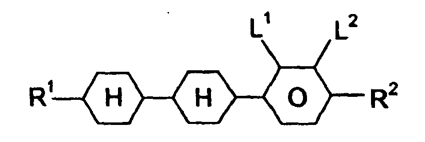 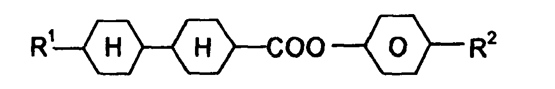 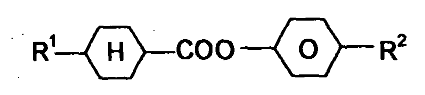 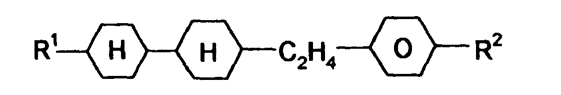 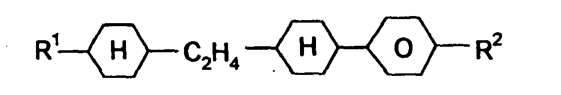 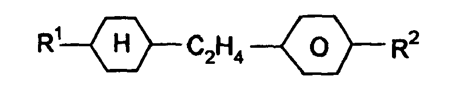 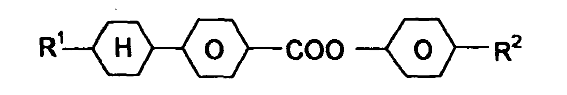 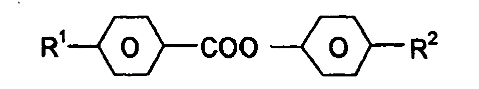 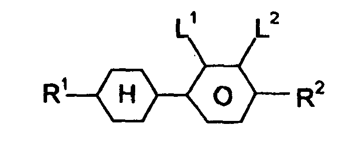 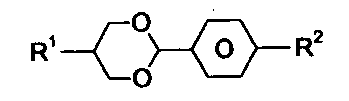 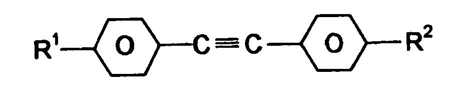 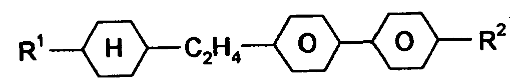 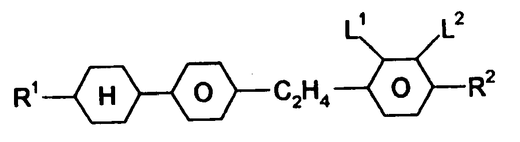 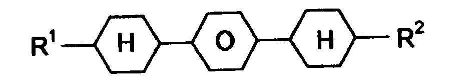 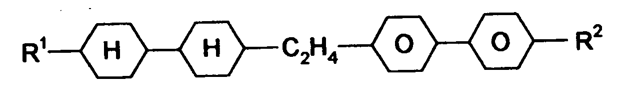 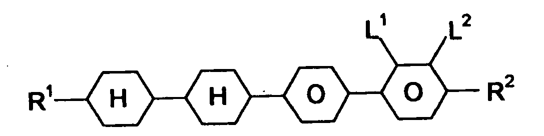 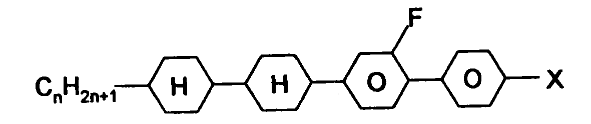 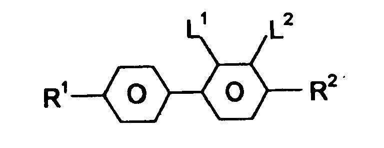 mit R¹, R² = -CₙH₂ₙ₊₁ oder -OCₙH₂ₙ₊₁ mit n = 1-8 und
L¹, L² = -H oder -F, 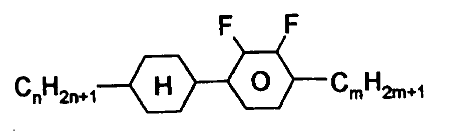 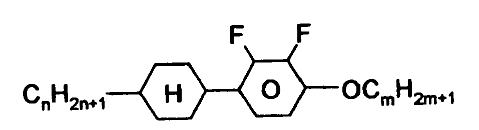 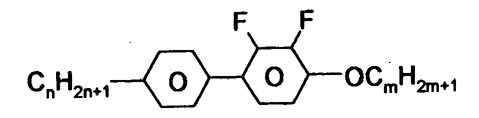 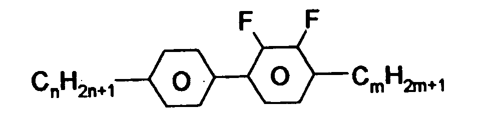 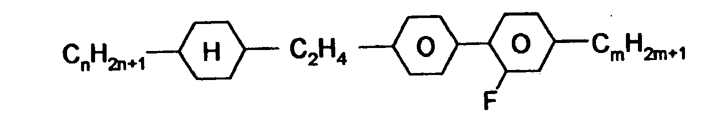 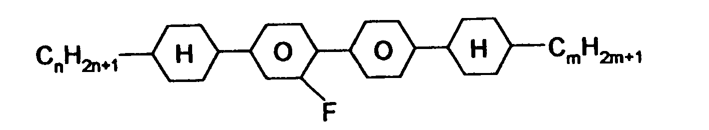 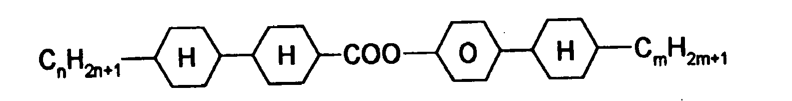 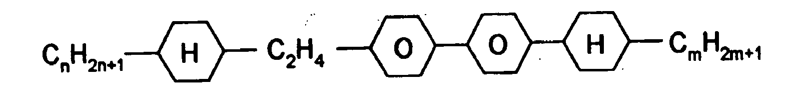 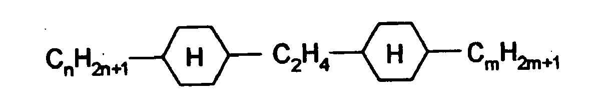 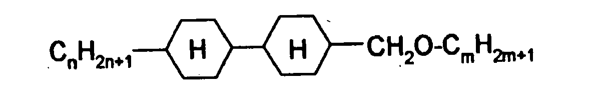 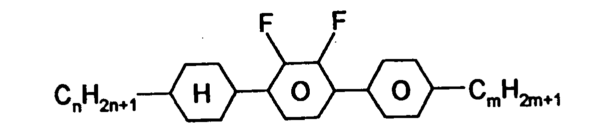 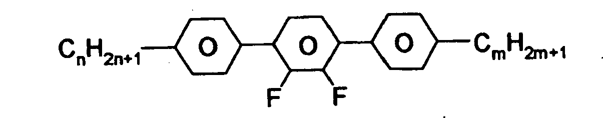 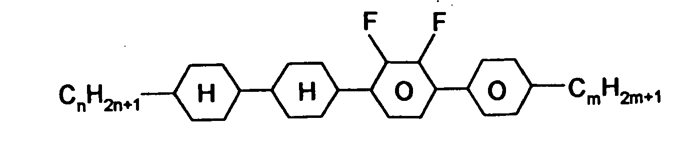 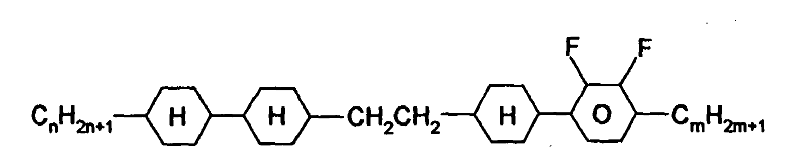 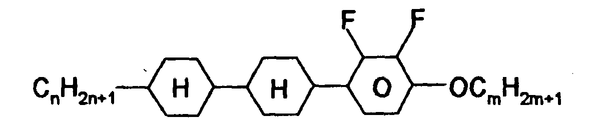 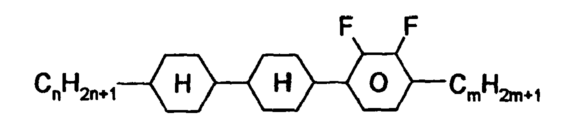 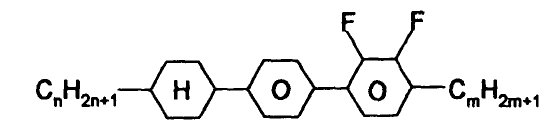 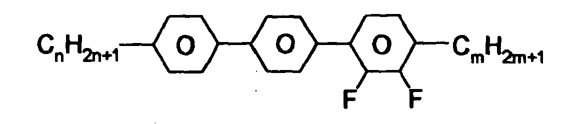 mit m,n=1-8.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, dass sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/ R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die Verbindungen der Formel (I) eignen sich wegen ihres negativen Δε für eine Verwendung in VA-TFT-Displays. Gegenstand der Erfindung ist daher auch eine elektrooptische Flüssigkristallanzeige, enthaltend ein erfindungsgemäßes flüssigkristallines Medium.

Die Erfindung wird anhand von Beispielen näher erläutert:

### Beispiele:

### Synthesebeispiele:

### Beispiel 1:

### 1-(2-Fluoro-6-iodo-4-methyl-phenyl)-2-(4`-propyl-bicyclohexyl-4-yl)-prop-2-en-1-ol

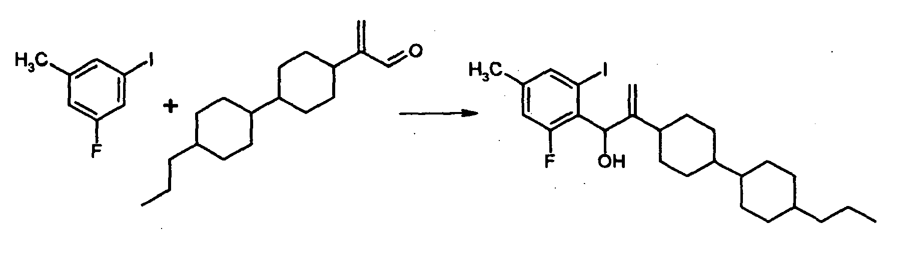

31,1 g (0,22 mol) 2,2,6,6-Tetramethylpiperidin werden in 350 ml Tetrahydrofuran vorgelegt und auf -20°C gekühlt. Bei dieser Temperatur werden 135 ml 1,6 M Butyllithium in Hexan (0,22 mol) zugetropft. Es wird auf -18°C gekühlt und bei dieser Temperatur 47,2 g (0,2 mol) 1-Fluor-3-iod-5-methylbenzol zugetropft. Man läßt eine Stunde bei -80°C nachrühren und gibt dann 52,5 g (0,2 mol) 3-(4'-Propyl-bicyclohexyl-4-yl)propenal zum Reaktionsgemisch. Man läßt auf 0°C aufwärmen, arbeitet nach Hydrolyse mit Wasser und verdünnter Salzsäure wie üblich auf.

Ausbeute 75 g (75 % d. Th.)

### Beispiel 2:

### 7-Fluor-5-methyl-2-(4`-propyl-bicyclohexyl-4-yl)-indan-1-on

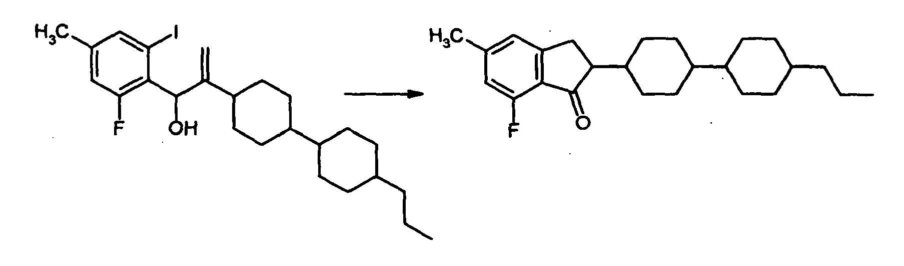

75 g (0,15 mol) 1-(2-Fluoro-6-iodo-4-methyl-phenyl)-2-(4'-propyl-bicyclohexyl-4-yl)-prop-2-en-1-ol, 50 ml Triethylamin, 400 mg Palladium(II)acetat (1,8 mmol) und 960 mg (3,7 mmol) Triphenylphosphin werden in 200 ml Acetonitril gelöst und über Nacht unter Rückfluß erhitzt. Nach Abkühlen auf Raumtemperatur wird in der üblichen Weise aufgearbeitet.

Ausbeute 44,5 g (80 % d. Th.)

### Beispiel 3:

### 4-Propyl-4'-(1,1,7-trifluoro-5-methyl-indan-2-yl)-bicyclohexyl

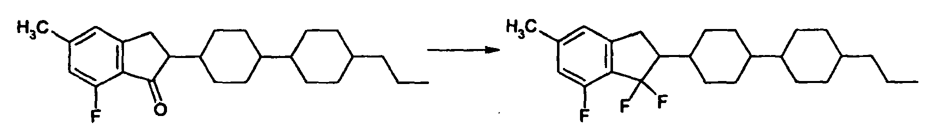

44,5 g (0,12 mol) 7-Fluor-5-methyl-2-(4'-propyl-bicyclohexyl-4-yl)-indan-1-on werden in 400 ml Dichlormethan gelöst und in einem Autoklaven bei Raumtemperatur mit 32,4 g (0,3 mol) SF₄ versetzt. Nach Beendigung der Reaktion wird in üblicher Weise aufgearbeitet.

Ausbeute 33,5 g (71 % d. Th.)

### Beispiel 4:

### 4'-(3,4-Difluoro-6-methyl-1H-inden-2-yl)-4-propyl-bicyclohexyl

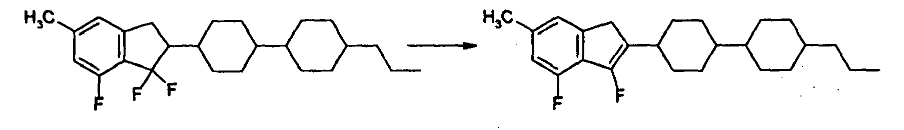

10,0 g (0,025 mol) 4-Propyl-4'-(1,1,7-trifluoro-5-methyl-indan-2-yl)-bicyclohexyl werden bei 60°C mit 5,6 g Kalium-tert.-butylat in 200 ml Tetrahydrofuran 6 Stunden gerührt. Anschließend wird in üblicher Weise aufgearbeitet.

Ausbeute 5,9 g (63 % d. Th.)

### Beispiel 5:

### 4'-(4-Fluoro-6-methyl-3-trifluoromethyl-1 H-inden-2-yl)-4-propyl-bicyclohexyl

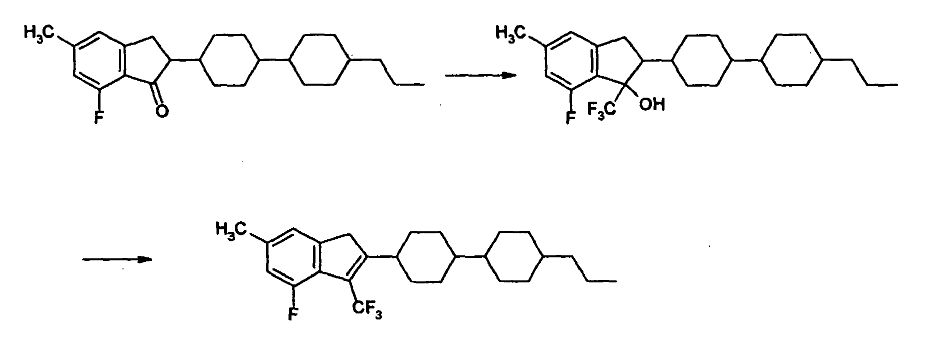

8,5 g (0,023 mol) 7-Fluor-5-methyl-2-(4'-propyl-bicyclohexyl-4-yl)-indan-1-on werden in 50 ml Tetrahydrofuran gelöst und auf 0°C abgekühlt. Nun werden 3,7 ml (0,025 mol) Trifluormethyltrimethylsilan zugegeben. Es werden 0,1 ml Tetrabutylammoniumfluorid (1 M Lösung in THF) zugegeben. Nach Beendigung der Reaktion wird in üblicher Weise aufgearbeitet und das Produkt anschließend in 40 ml Methanol aufgenommen, mit 200 mg Kaliumfluorid versetzt und 10 Stunden unter Rückfluß erhitzt. Das entstandene Carbinol wird in 30 ml Pyridin gelöst und tropfenweise mit 1,7 ml (0,024 mol) Thionylchlorid versetzt. Die Mischung wird für 10 Stunden bei Raumtemperatur gerührt und anschließend in üblicher Weise aufgearbeitet.

Ausbeute 6,9 g (71 % d. Th.)

### Beispiel 6:

### 2-Iod-4-methyl-6-fluorphenol

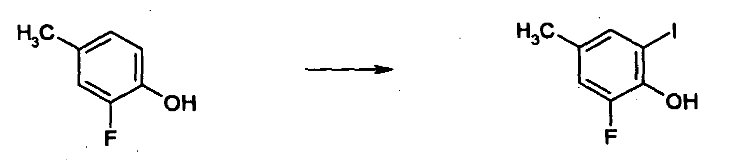

55,5 g (0,44 mol) 2-Fluor-4-methylphenol und 120,3 g (0,87 mol) Kaliumcarbonat werden in 275 ml Wasser gelöst und auf 5°C gekühlt. Dann werden portionsweise 127,5 g Iod (0,5 mol) eingetragen. Nach Beendigung der Reaktion wird in üblicher Weise aufgearbeitet.

Ausbeute 86,5 g (78 % d. Th.)

### Beispiel 7:

### 7-Fluor-5-methyl-2-(4'-propyl-bicyclohexyl-4-yl)-benzofuran

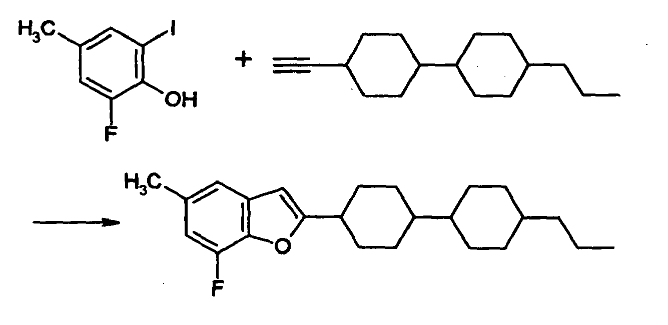

50,5 g (0,2 mol) 2-Iod-4-methyl-6-fluorphenol, 4,2 g (6 mmol) Bis(triphenylphosphin)paliadium(II)chlorid, 1,2 g (6,3 mmol) Kupfer(I)iodid, 40,5 g (0,4 mol) Triethylamin werden in 200 ml Dimethylformamid vorgelegt und bei Raumtemperatur 46,5 g (0,2 mol) 4'-propyl-bicyclohexylacetylen, gelöst in Dimethylformamid, zugetropft. Man läßt eine Stunde bei

Raumtemperatur und 2 Stunden bei 40°C nachrühren. Anschließend wird in üblicher Weise aufgearbeitet.

Ausbeute 38,5 g (54 % d. Th.)

Durch Hydrieren bei Normaldruck unter Verwendung von Palladium auf Kohle (10 %) kann die Verbindung zum Dihydrobenzofuran umgesetzt werden.

### Beispiel 8:

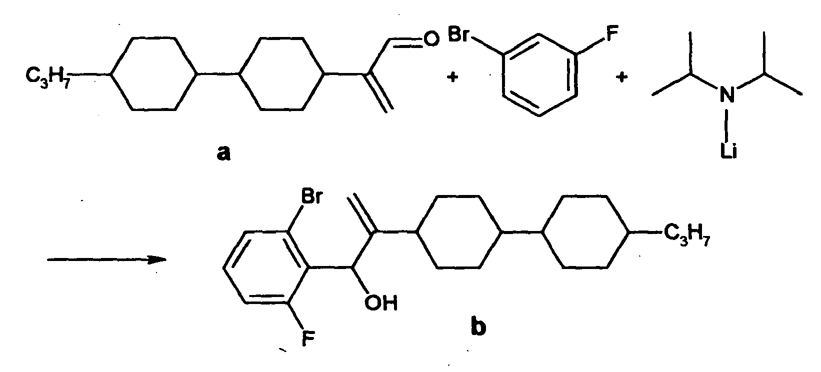

41,0 ml einer 2-molaren Lösung von Lithiumdiisopropylamid in Cyclohexan/Ethylbenzol/Tetrahydrofuran (79,612 mmol) und 150 ml THF werden vorgelegt und bei -74°C mit 60,3 g (93,143 mmol) 1-Brom-3-fluorbenzol in 20 ml THF versetzt. Es wird 1 Stunde gerührt und anschließend eine Lösung von 18,4 g (70,111 mmol) a in 40 ml THF zugesetzt. Es wird 12 Stunden gerührt, mit 1 n-HCL angesäuert und mit Methyl-t.-butyl-ether extrahiert. Die organische Phase wird getrocknet, eingeengt und das Produkt wird aus n-Hexan kristallisieren gelassen.

Ausbeute 25,9 g (84,4 % d. Th.)

### Beispiel 9:

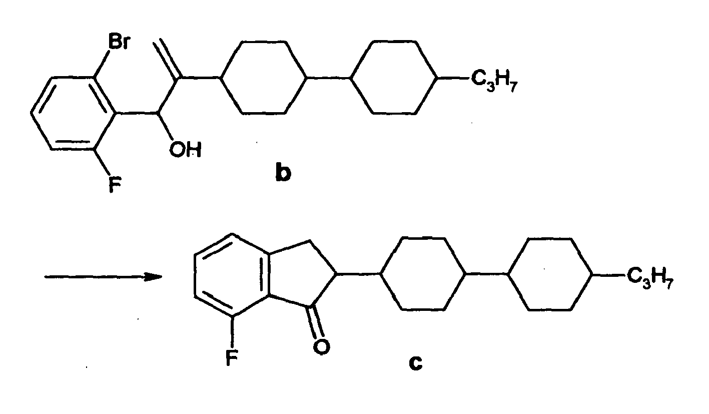

95,8 g (58,98 mmol) **b** werden in 185 ml Acetonitril und 40 ml Triethylamin in der Wärme gelöst. Zu dieser Lösung werden 2,5 g (2,538 mmol) Bis-(tri-o-totylphosphin)palladium(II)chlorid als Katalysator zugegeben. Es wird unter Stickstoff auf 90°C erwärmt. Anschließend wird wie üblich aufgearbeitet. Kristallisation aus Hexan ergibt 16,2 g **c**.

Ausbeute 16,2 g (77,0 % d. Th.)

### Beispiel 10

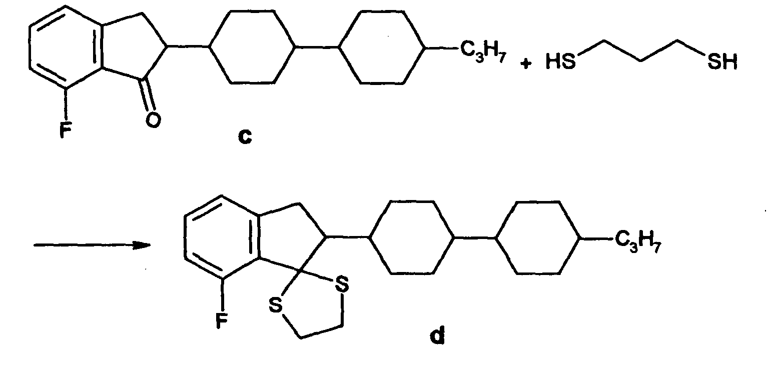

5,9 g (16,548 mmol) c und 3,207 mml (32,0 mmol) 1,3-Propandithiol werden in 50 ml Dichlormethan gelöst. Zu dieser Lösung werden bei -10°C 10 ml (79,617 mmol) Bortrifluorid-Diethylether-Komplex zugegeben. Es wird bei einer Temperatur von -5 bis -10°C weiter gerührt und anschließend über Nacht auftauen gelassen. Die Produktlösung wird auf Hydrogencarbonat gegeben, und es wird bis zum Ende der Gasentwicklung gerührt. Anschließend wird zweimal mit Dichlormethan extrahiert, getrocknet und mit einem Methyl-t.-butylether/Heptan 1:10-Gemisch chromatographiert.

### Beispiel 11:

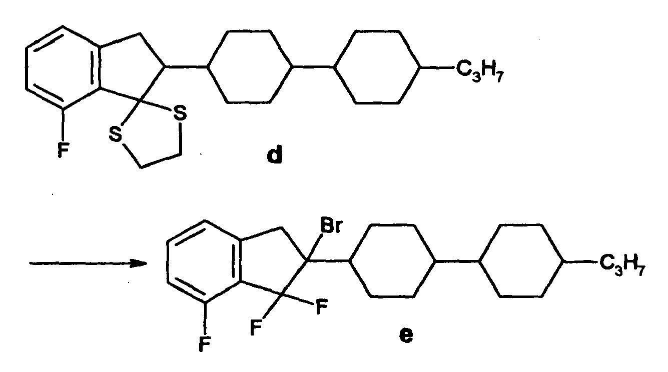

41 ml einer 65 %igen Lösung von Fluorwasserstoff in Pyridin und 11,4 g (39,87mmol) 1,3-Dibrom-5,5-dimethylhydantoin werden in 35 ml Dichlormethan vorgelegt und auf -75°C abgekühlt. Zu dieser Lösung werden 4,1 g (9,178 mmol) d in 25 ml Dichlormethan zugegeben. Die Kühlung wird entfernt und der Ansatz über Nacht gerührt. Anschließend werden 200 ml eisgekühlte Natriumhydrogensulfit-Lösung und 500 ml 2 n-NaOH zugegeben. Die wässrige Phase wird dreimal mit Dichlormethan extrahiert, die organische Phase mit gesättigter Natriumchlorid-Lösung gewaschen, getrocknet und eingeengt. Das Produkt wird mit Diethylether/Heptan 1 : 20 chromatographiert.

### Beispiel 12:

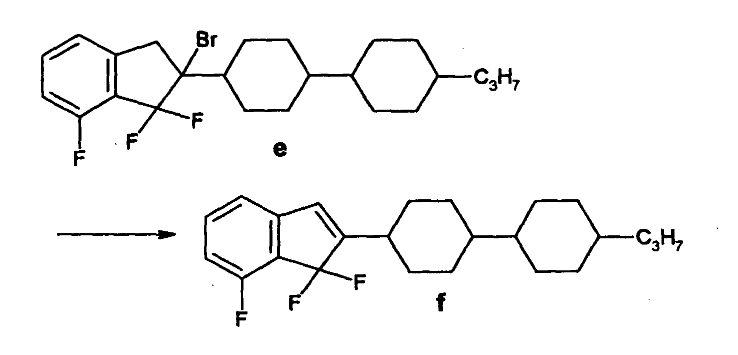

4,20 g des Rohproduktes aus Beispiel 12 werden mit 8 ml Diazabicyclo[5.4.0]undec-7-en in 8 ml THF versetzt. Es wird bei Raumtemperatur bis zur vollständigen Umsetzung (DC-Kontrolle) gerührt. Anschließend wird eingeengt, mit Wasser und Dichlormethan aufgenommen, extrahiert, getrocknet und mit Hexan chromatographiert.

### Beispiel 13:

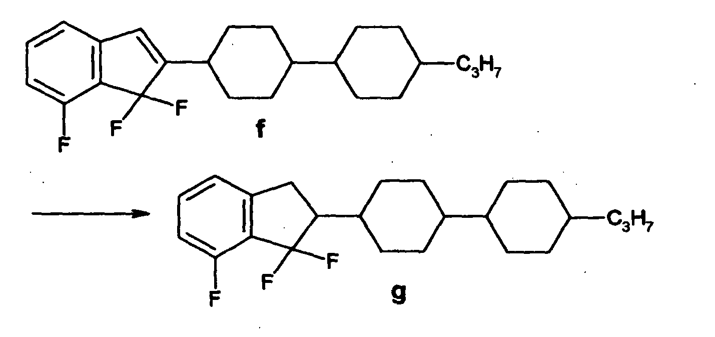

2,3 g des Rohproduktes aus Beispiel 12 werden an 1,8 g 5%igem Palladium auf Kohle in 30 ml Tetrahydrofuran hydriert.

Analog den Beispielen 8-13 werden die nachstehenden Verbindungen erhalten:

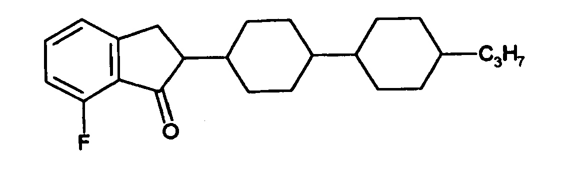

| | |
|---|---|
| Phasen: | K 122 |
| Kp: | 23,7°C |
| Δε: | -8,5 |
| Δn: | 0,075 |

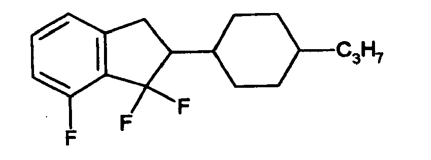

| | |
|---|---|
| Phasen: | K 99 I |
| Kp: | 18,1°C |
| Δε: | -7,1 |
| Δn: | 0,086 |

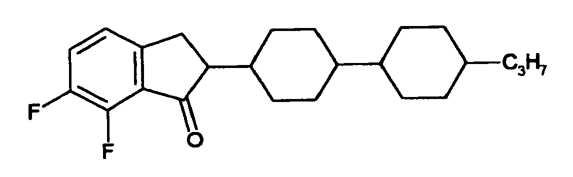

| | |
|---|---|
| Phasen: | K 118 I |
| Kp: | 50,5°C |
| Δε: | -11,4 |
| Δn: | 0,065 |

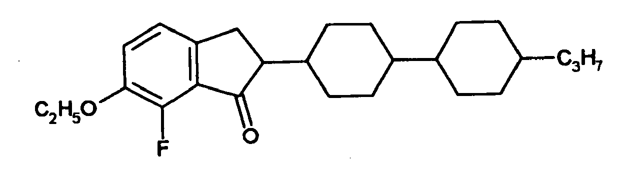

| | |
|---|---|
| Phasen: | K 134 I |
| Kp: | 104,3°C |
| Δε: | -11,7 |
| Δn: | 0,0775 |

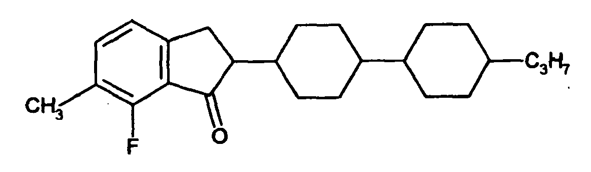

| | |
|---|---|
| Phasen: | K 105 I |
| Kp: | 77,7°C |
| Δε: | -8,2 |
| Δn: | 0,077 |

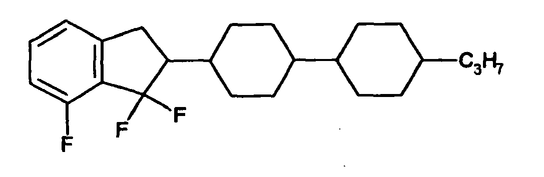

| | |
|---|---|
| Phasen: | K 171 SmB (170) N(170,7) I |
| Kp: | 180,5°C |
| Δε: | -5,4 |
| Δn: | 0,0754 |

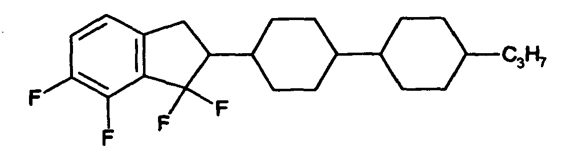

| | |
|---|---|
| Phasen: | K 130 SmB 168 N 203,7 I |
| Kp: | 202,3°C |
| Δε: | -8,3 |
| Δn: | 0,075 |

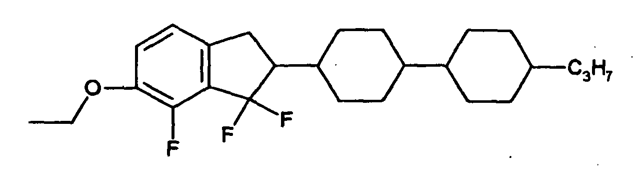

| | |
|---|---|
| Phasen: | K 109 SmA-1 156 SmA-2 183 N 220,7 **I** |
| Kp: | 253,5°C |
| Δε: | -6,7 |
| Δn: | 0,087 |

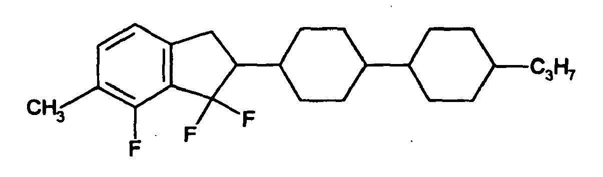

| | |
|---|---|
| Phasen: | K 110 SmA-1 168 SmA-2 171 N 207,0 I |
| Kp: | 233,3°C |
| Δε: | -5,6 |
| Δn: | 0,0765 |

Vorstehend bedeuten Kp den Klärpunkt, Δn die dielektrische Anisotropie und Δn die Doppelbrechung.

### Mischungsbeispiele:

Folgende Abkürzungen werden verwendet:

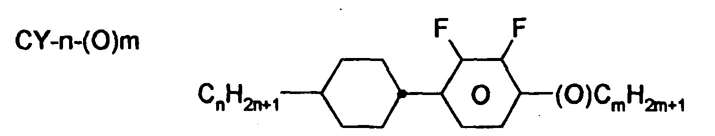

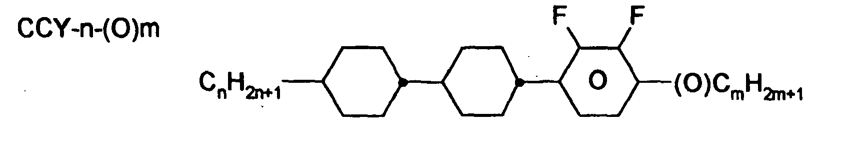

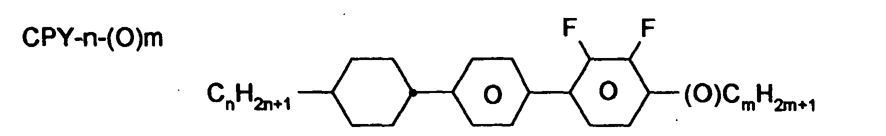

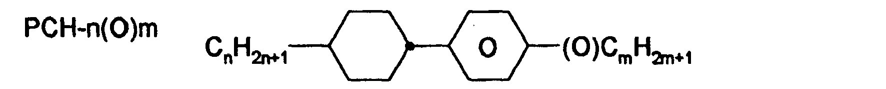

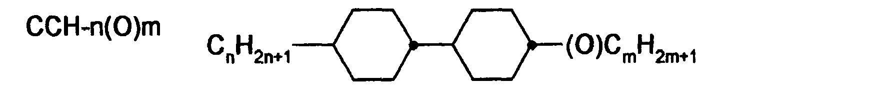

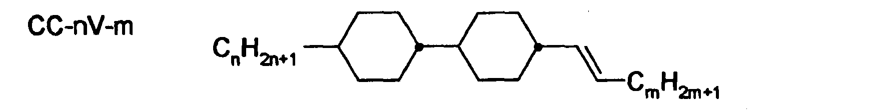

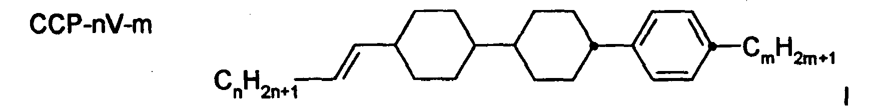

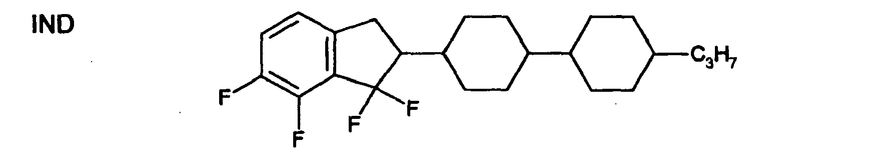

### Weiterhin bedeuten:

- Kp: Klärpunkt [°C]
- Δn: optische Anisotropie (Doppelbrechung) bei 20°C und 589 nm
- Δε: dielektrische Anisotropie bei 20°C und 1 kHz
- ε∥: Dielektrizitätskonstante parallel zum Direktor bei 20°C und 1 kHz
- K₃/K₁: Verhältnis der elastischen Konstanten k₃ und K₁
- γ₁: Rotationsviskosität [mPa˙s] (bei 20°C, sofern nicht anders angegeben)
- V₀: kapazitive Schwellenspannung [V]

Die zur Messung der kapazitiven Schwellenspannung verwendete Anzeige weist zwei planparallele Trägerplatten im Abstand von 20 µm und Elektrodenschichten mit darüberliegenden Orientierungsschichten aus geriebenem Polyimid auf den Innenseiten der Trägerplatten auf, welche eine homöotrope Randorientierung der Flüssigkristallmoleküle bewirken.
Die polymerisierbaren Verbindungen werden in der Anzeige durch UV-Bestrahlung mit einer Stärke von 28 mW/cm² und einer Dauer von ca. 2 Minuten polymerisiert, wobei gleichzeitig eine Spannung von 10 V an die Anzeige gelegt wird.

Es wurden die nachstehenden Flüssigkristallmischungen bereitet und daran die nachstehenden Werte bestimmt.

### Beispiel 14:

| | | | |
|---|---|---|---|
| CY-3-O2 | 20.00 % | Kp. | +74,0 |
| CY-5-O2 | 11.00 % | Δn | 0,0813 |
| CCY-3-O3 | 10.00% | Δε | -3,9 |
| CCY-4-O2 | 10.00 % | εₗₗ | 3,7 |
| CPY-2-O2 | 7.00% | K₃/K₁ | 1,04 |
| CC-5-V | 20.00 % | γ₁ | 109 |
| CC-3-V1 | 12.00 % | V₀ | 2,02 |
| CCH-35 | 5.00% | | |
| IND | 5.00 % | | |

### Beispiel 15:

| | | | |
|---|---|---|---|
| CY-3-O2 | 12.00% | Kp. | + 75,0 |
| CY-5-O2 | 12.00% | Δn | 0,0823 |
| CCY-4-O2 | 7.00% | Δε | -3,2 |
| CPY-2-O2 | 12.00 % | εₗₗ | 3,5 |
| CPY-3-O2 | 3.00 % | K₃/K₁ | 0,97 |
| CC-5-V | 20.00 % | γ₁ | 91 |
| CC-3-V1 | 12.00 % | V₀ | 2,17 |
| CC-4-V | 10.00 % | | |
| CCH-35 | 4.00% | | |
| IND | 8.00 % | | |

## Patentansprüche

1. Indane mit negativem Δε der Formel (Ia) oder (Ib) 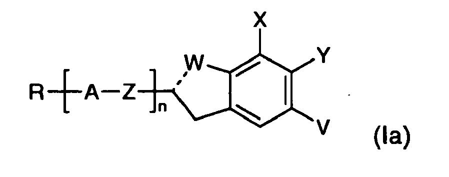 oder 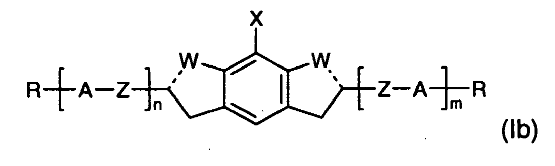 wobei bedeutet:
R jeweils unabhängig voneinander einen unsubstituierten, einen einfach durch -CF₃ oder einen mindestens einfach durch Halogen substituierten Alkyl- oder Alkoxyrest mit 1 bis 12 C-Atomen, einen Oxaalkyl-, Alkenyl- oder Alkenyloxyrest mit 2 bis 12 C-Atomen oder einen Oxaalkenylrest mit 3 bis 12 C-Atomen, wobei in diesen Resten auch eine oder mehrere CH₂-Gruppen jeweils unabhängig voneinander durch -O-, -S-, -CO-, -COO-, -OCO- oder -OCO-O- so ersetzt sein können, daß Heteroatome nicht direkt mit einander verknüpft sind,
A jeweils unabhängig voneinander 1,4-Phenylen, worin =CH- ein- oder zweimal durch =N- ersetzt sein kann, und das ein- bis viermal unabhängig voneinander mit Halogen (-F, -Cl, -Br, -l), -CN, -CH₃,-CH₂F, -CHF₂, -CF₃, -OCH₃, -OCH₂F, -OCHF₂ oder -OCF₃ substituiert sein kann, 1,4-Cyclohexylen, 1,4-Cyclohexenylen oder 1,4-Cyclohexadienylen, worin -CH₂- ein- oder zweimal unabhängig voneinander durch -O- oder -S- ersetzt sein kann, und die ein- oder mehrfach durch Halogen substituiert sein können,
Z jeweils unabhängig voneinander eine Einfachbindung, eine -CH₂-CH₂-, -CF₂CF₂- -CH=CH-, -CF=CH-, -CH=CF-, -C=C-, -CO-O-, -O-CO-, -O-CH₂-, -CH₂-O-, O-CF₂- oder eine -CF₂-O- -Gruppe,
X -F,
Y, V jeweils unabhängig voneinander Wasserstoff, einen unsubstituierten, einen einfach durch -CF₃ oder mindestens einfach durch Halogen substituierten Alkylrest, Alkoxyrest, Alkenylrest oder Alkinylrest mit 1 bis 15 bzw. 2 bis 15 C-Atomen, wobei in diesen Resten auch eine oder mehrere CH₂-Gruppen durch -O-, -S-, -CO-, -COO-, -OCO- oder -OCO-O- so ersetzt sein können, dass Heteroatome nicht direkt benachbart sind,
Y zusätzlich auch -F oder -Cl,
W jeweils unabhängig voneinander -O-, -C(O)-, -CHF- oder -CF₂- bzw. -CF= und zusätzlich auch -CH₂- in Formel (Ib),
n, m jeweils unabhängig voneinander 1 oder 2,
und die gepunktete Linie für eine Einfach- oder eine Doppelbindung steht, mit der Maßgabe, dass die Verbindung der Formel (la), worin R=Propyl,A=1,4-Cyclohexylen, Z = eine Einfachbindung, X = -F, Y und V = Wasserstoff, W =-C(O)- und n = 1 ausgenommen ist.

2. Indane nach Anspruch 1 der Formel (la).

3. Indane nach Anspruch 1 oder 2, worin W jeweils unabhängig voneinander -O-, -C(O)-, -C(F)= oder -CF₂- ist.

4. Indane nach Anspruch 1:

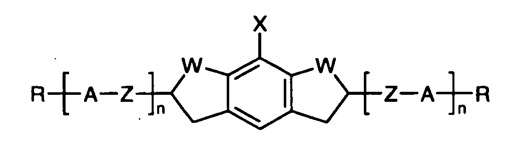

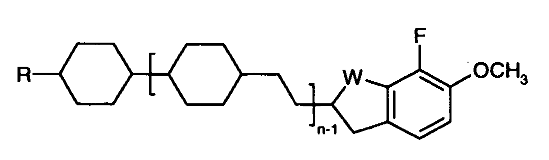

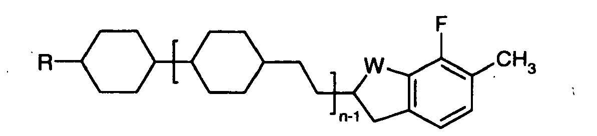

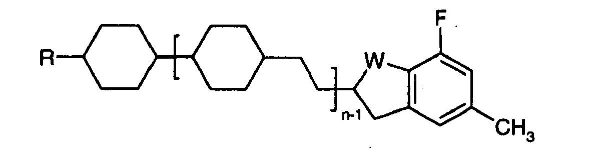

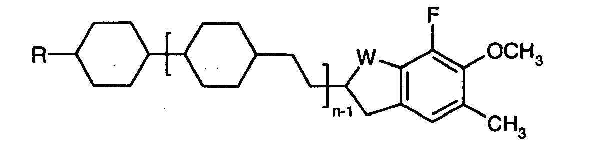

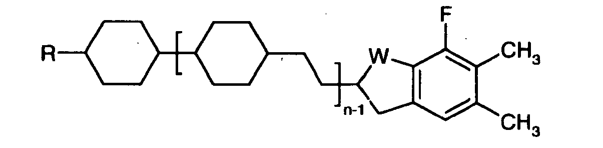

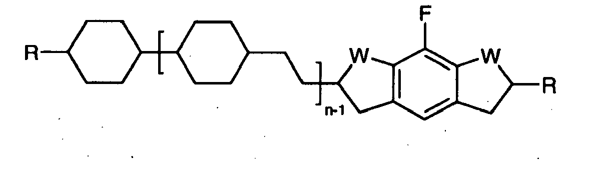

5. Indane nach Anspruch 1:

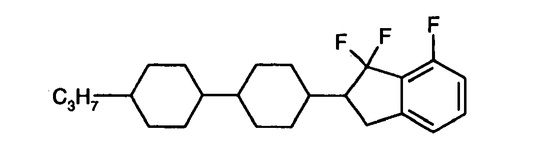

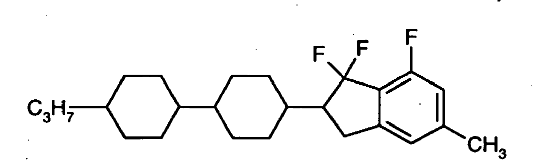

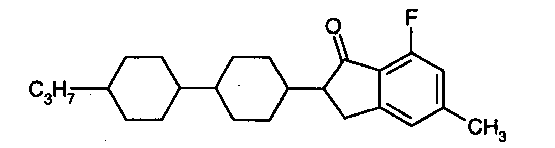

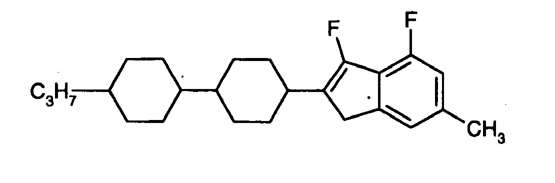

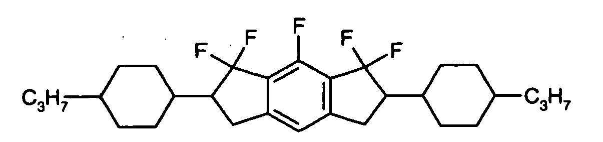

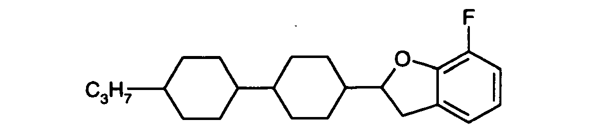

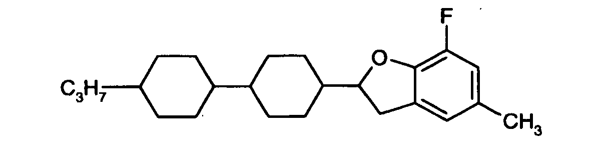

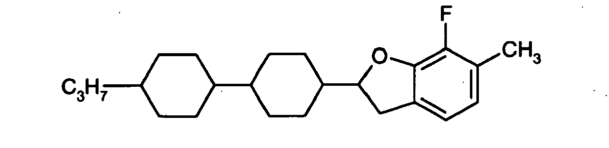

6. Flüssigkristallines Medium mit mindestens zwei flüssigkristallinen Verbindungen umfassend mindestens eine Verbindung der Formel (la) und/oder (Ib) gemäß Anspruch 1.

7. Elektrooptische Flüssigkristallanzeige, enthaltend ein flüssigkristallines Medium nach Anspruch 6.

## Claims

1. Indanes having negative Δε of the formula (Ia) or (Ib) 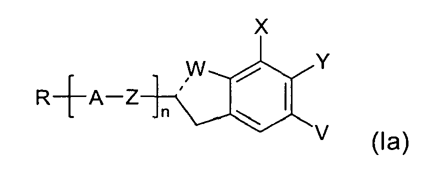 or 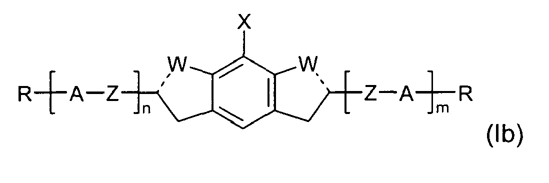 in which:
R, in each case independently of one another, is an alkyl or alkoxy radical having 1 to 12 carbon atoms which is unsubstituted, monosubstituted by -CF₃ or at least monosubstituted by halogen, an oxaalkyl, alkenyl or alkenyloxy radical having 2 to 12 carbon atoms or an oxaalkenyl radical having 3 to 12 carbon atoms, where one or more CH₂ groups in these radicals may also, in each case independently of one another, be replaced by -O-, -S-, -CO-, -COO-, -OCO- or -OCO-O- in such a way that heteroatoms are not linked directly to one another,
A, in each case independently of one another, is 1,4-phenylene, in which =CH- may be replaced once or twice by =N- and which may be mono- to tetrasubstituted, independently of one another, by halogen (-F, -Cl, -Br, -I), -CN, -CH₃, -CH₂F, -CHF₂, -CF₃, -OCH₃, -OCH₂F, -OCHF₂ or -OCF₃, 1,4-cyclohexylene, 1,4-cyclohexenylene or 1,4-cyclohexadienylene, in which -CH₂- may be replaced once or twice by, independently of one another, -O- or-S- and which may be mono- or polysubstituted by halogen,
Z, in each case independently of one another, is a single bond, a -CH₂-CH₂-, -CF₂CF₂- -CH=CH-, -CF=CH-, -CH=CF-, -C≡C-, -CO-O-, -O-CO-, -O-CH₂-, -CH₂-O-, O-CF₂- or a -CF₂-O- group,
X is -F,
Y, V are each, independently of one another, hydrogen, an alkyl, alkoxy, alkenyl or alkinyl radical having 1 to 15 or, respectively, 2 bis 15 carbon atoms which is unsubstituted, monosubstituted by -CF₃ or at least monosubstituted by halogen, where one or more CH₂ groups in these radicals may also, in each case independently of one another, be replaced by -O-, -S-, -CO-, -COO-, -OCO- or -OCO-O- in such a way that heteroatoms are not linked directly to one another,
Y is additionally -F or -Cl,
W, in each case independently of one another, is -O-, -C(O)-, -CHF- or -CF₂- or -CF= and, in formula (Ib), additionally -CH₂-
n, m are each, independently of one another, 1 or 2,
and the dotted line is a single bond or a double bond, with the proviso that the compound of the formula (Ia) in which R=propyl, A=1,4-cyclohexylene, Z=a single bond, X=-F, Y and V=hydrogen, W=-C(O)- and n=1 is excluded.

2. Indanes according to claim 1 of the formula (Ia).

3. Indanes according to claim 1 or 2, in which W, in each case independently of one another, is -O-, -C(O)-, -C(F)= or -CF₂-.

4. Indanes according to claim 1:

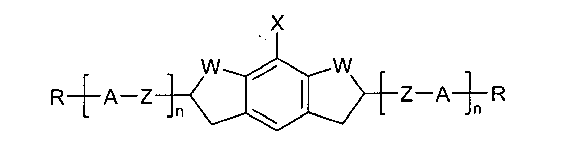

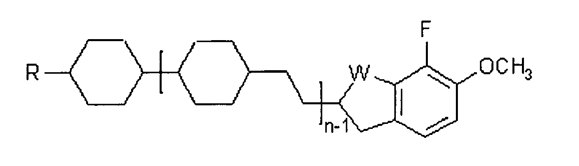

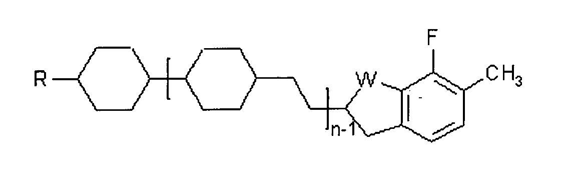

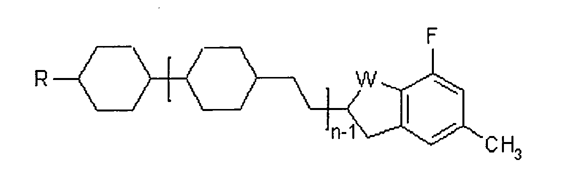

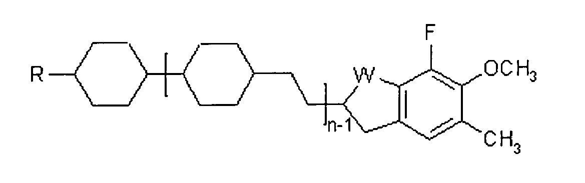

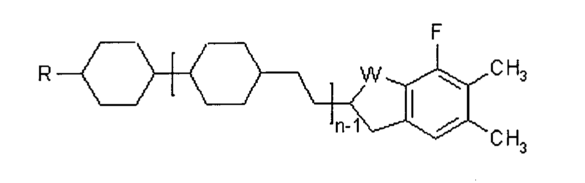

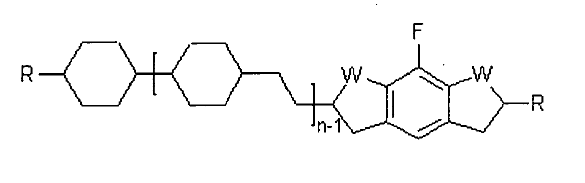

5. Indanes according to claim 1:

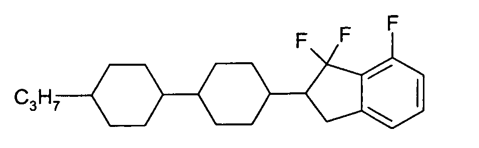

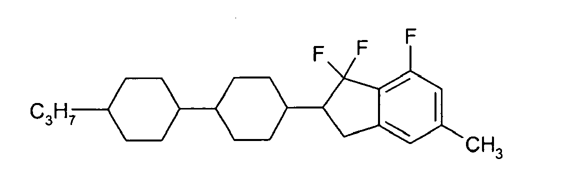

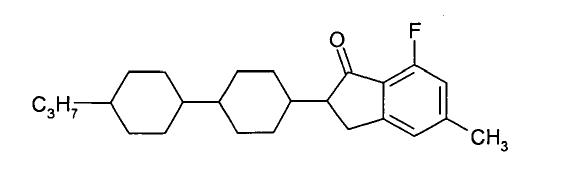

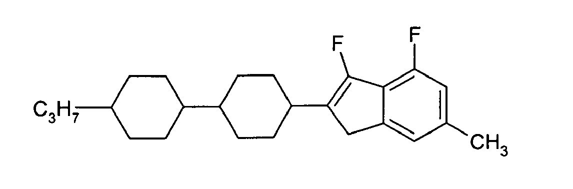

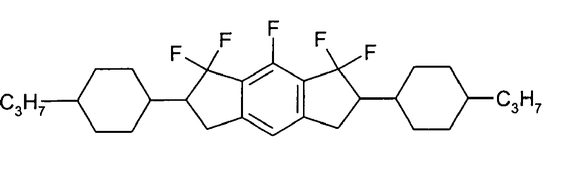

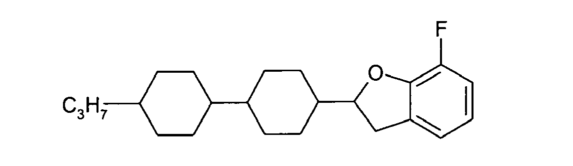

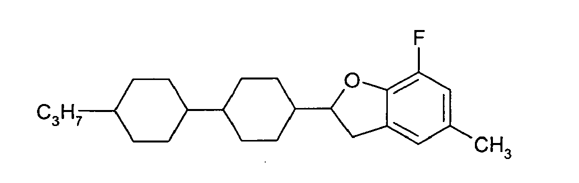

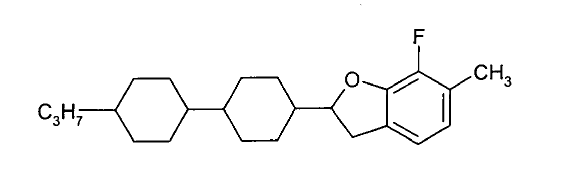

6. Liquid-crystalline medium comprising at least two liquid-crystalline compounds including at least one compound of the formula (Ia) and/or (Ib) according to claim 1.

7. Electro-optical liquid-crystal display comprising a liquid-crystalline medium according to claim 6.

## Revendications

1. Indanes à Δε négatif de formule (Ia) ou (Ib) 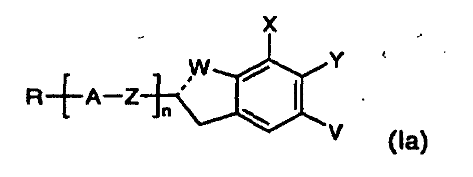 ou 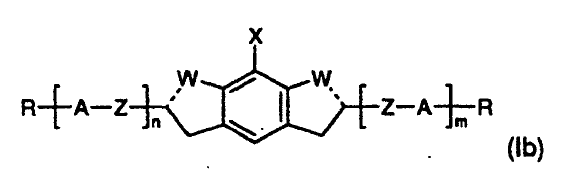 formules dans lesquelles :
R représente, chaque fois indépendamment, un radical alkyle ou alcoxy non substitué, monosubstitué par -CF₃ ou au moins une fois substitué par halogène, ayant de 1 à 12 atomes de carbone, un radical oxaalkyle, alcényle ou alcényloxy ayant de 2 à 12 atomes de carbone, ou un radical oxaalcényle ayant de 3 à 12 atomes de carbone, un ou plusieurs groupes CH₂ dans ces radicaux pouvant également être remplacés chacun, indépendamment les uns des autres, par -O-, -S-, -CO-, -COO-, -OCO- ou -OCO-O-, de manière que les hétéroatomes ne soient pas directement liés entre eux,
A représente, chaque fois indépendamment, un groupe 1,4-phénylène, dans lequel =CH- peut être une ou deux fois remplacé par =N-, et qui peut être une à quatre fois substitué, chaque fois indépendamment, par halogène (-F, -Cl, -Br, -I), -CN, -CH₃, -CH₂F, -CHF₂, -CF₃, -OCH₃, -OCH₂F, -OCHF₂ ou -OCF₃, un groupe 1,4-cyclohexylène, 1,4-cyclohexénylène ou 1,4-cyclohexadiénylène, dans lesquels -CH₂- peut être remplacé une ou deux fois, indépendamment l'une de l'autre, par -O- ou -S-, et qui peuvent être une ou plusieurs fois substitués par halogène,
Z représente, chaque fois indépendamment, une liaison simple, un groupe -CH₂-CH₂-, -CF₂CF₂-, -CH=CH-, -CF=CH-, -CH=CF-, -C≡C-, -CO-O-, -O-CO-, -O-CH₂-, -CH₂-O-, O-CF₂- ou un groupe -CF₂-O-,
X représente -F,
Y, V représentent chacun, indépendamment l'un de l'autre, un atome d'hydrogène, un radical alkyle, alcoxy, alcényle ou alcynyle non substitué, monosubstitué par -CF₃ ou au moins une fois substitué par halogène, ayant de 1 à 15 ou, respectivement, de 2 à 15 atomes de carbone, un ou plusieurs groupes CH₂ dans ces radicaux pouvant également être remplacés par -O-, -S-, -CO-, -COO-, -OCO- ou -OCO-O-, de manière que les hétéroatomes ne soient pas directement liés entre eux,
Y représente en outre également -F ou -Cl,
W représente, chaque fois indépendamment des autres, -O-, -C(O)-, -CHF- ou -CF₂- ou -CF= et en outre également -CH₂- dans la formule (Ib),
n, m représentent chacun, indépendamment l'un de l'autre, 1 ou 2,
et le pointillé représente une liaison simple ou une double liaison, étant entendu que le composé de formule (Ia) dans lequel R = propyle, A = 1,4-cyclohexylène, Z représente une liaison simple, X = -F, Y et V représentent un atome d'hydrogène, W = -C(O)- et n = 1 est exclu.

2. Indanes selon la revendication 1, de formule (Ia).

3. Indanes selon la revendication 1 ou 2, dans lesquels W représente, chaque fois indépendamment des autres, -O-, -C (O) -, -C (F) = ou -CF₂- .

4. Indanes selon la revendication 1 :

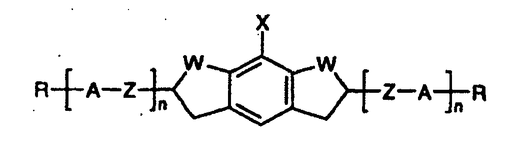

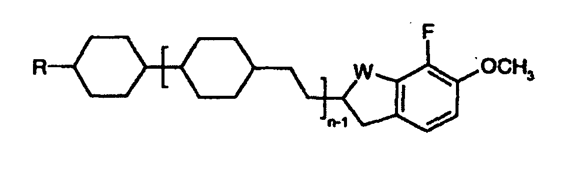

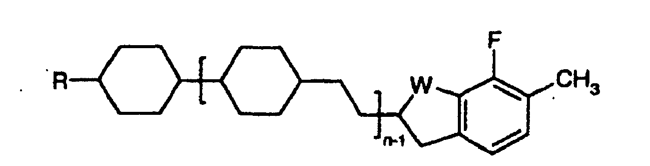

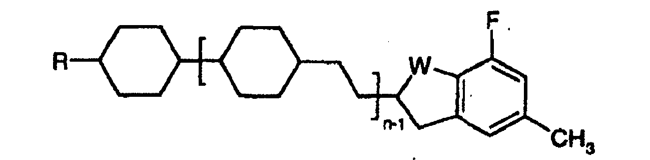

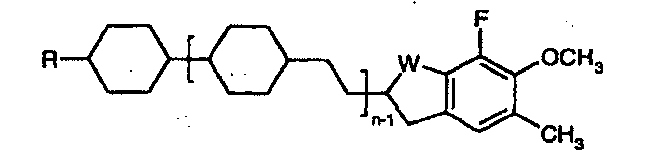

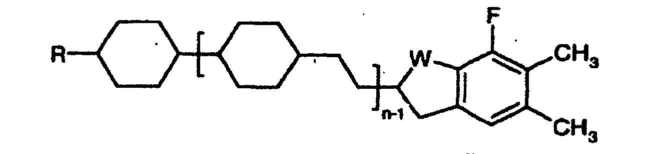

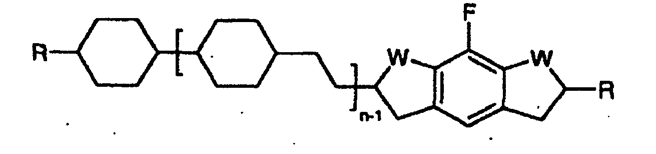

5. Indanes selon la revendication 1 :

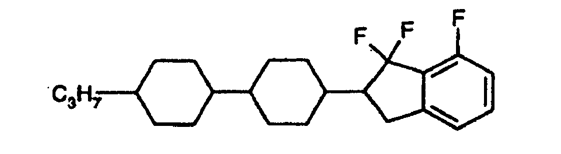

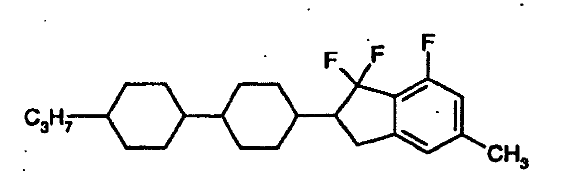

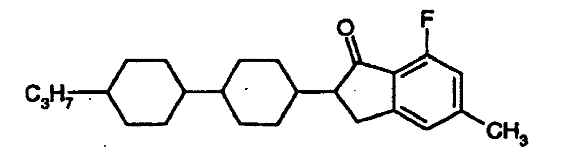

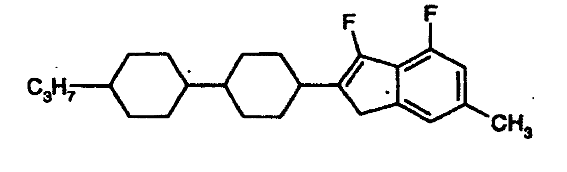

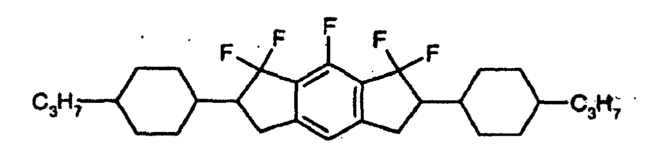

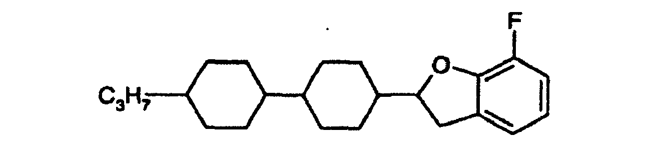

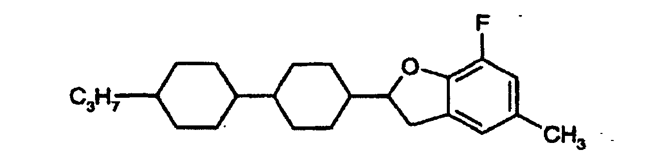

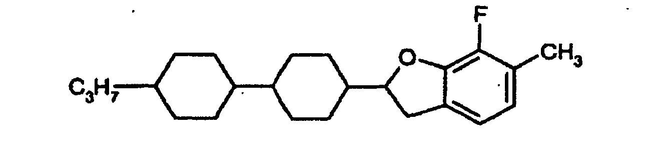

6. Milieu à cristaux liquides, comportant au moins deux composés de type cristal liquide comprenant au moins un composé de formule (Ia) et/ou de formule (Ib) selon la revendication 1.

7. Afficheur électro-optique à cristaux liquides, contenant un milieu à cristaux liquides selon la revendication 6.
